# EUROPEAN PATENT APPLICATION

(11) **EP 4 130 193 A1**
(43) Date of publication of application: **08.02.2023**
(21) Application number: 21780339.4
(22) Date of filing: 18.03.2021
(51) Int. Cl.: C09K 11/06, H05B 33/10, H01L 51/50

(54) **ORGANIC ELECTROLUMINESCENT ELEMENT**

(30) Priority: 31.03.2020 JP 2020063315
(71) Applicant: NIPPON STEEL Chemical & Material Co., Ltd., Tokyo 103-0027 (JP)
(72) Inventor: HAYASHI, Kentaro, Tokyo 103-0027 (JP); OGAWA, Junya, Tokyo 103-0027 (JP); IKENAGA, Yuji, Tokyo 103-0027 (JP)
(74) Representative: Vossius & Partner Patentanwälte Rechtsanwälte mbB
(86) International application number: PCT/JP2021/011250
(87) International publication number: WO 2021/200243

(57) **Abstract**

To provide a practically useful organic electroluminescent device which is improved in luminous efficiency and at the same time, sufficiently secures the stability during driving. An organic electroluminescent device, wherein a light-emitting layer includes two host materials different from each other, and a dopant material, one of the host materials is a compound represented by general formula (1) and the other of the host materials is a compound represented by general formula (2). A ring A is a heterocycle represented by formula (1a), X represents N or C-Ar', Y represents O, S, N-Ar³, or C-Ar⁴Ar⁵, and any one of Z¹ to Z⁴ represents a carbon atom binding to a 6-membered ring containing X and the others and Z⁵ to Z⁸ each represent C-Ar' or N. Ar, Ar' and Ar¹ to Ar⁵ each represent, for example, hydrogen, or an alkyl group having 1 to 20 carbon atoms. A ring B is a heterocycle represented by formula (2a), and Ar⁶ and Ar⁷ each represent, for example, hydrogen, or an alkyl group having 1 to 20 carbon atoms.

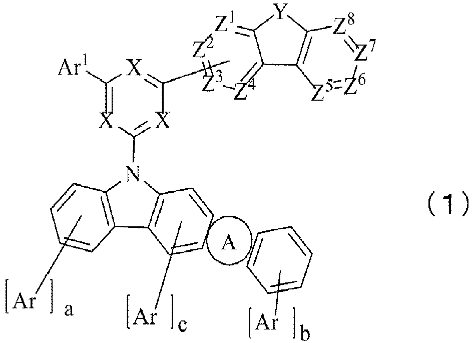

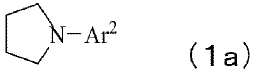

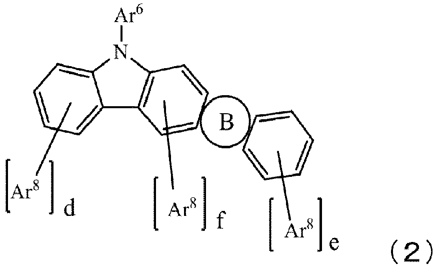

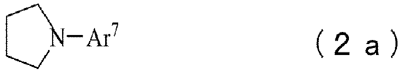

## Description

### Technical Field

The present invention relates to an organic electroluminescent element or device (hereinafter referred to as an organic EL device), more specifically relates to an organic EL device comprising a specific mixed host material.

### Background Art

Application of a voltage to an organic EL device allows injection of holes and electrons from an anode and a cathode, respectively, into a light-emitting layer. Then, in the light-emitting layer, injected holes and electrons recombine to generate excitons. At this time, according to statistical rules of electron spins, singlet excitons and triplet excitons are generated at a ratio of 1:3. Regarding a fluorescence-emitting organic EL device using light emission from singlet excitons, it is said that the internal quantum efficiency thereof has a limit of 25%. Meanwhile, regarding a phosphorescent organic EL device using light emission from triplet excitons, it is known that intersystem crossing is efficiently performed from singlet excitons, the internal quantum efficiency is enhanced to 100%.

Further, highly efficient organic EL devices utilizing delayed fluorescence have been developed recently. For example, Patent Literature 1 discloses an organic EL device utilizing a TTF (Triplet-Triplet Fusion) mechanism, which is one of delayed fluorescence mechanisms. The TTF mechanism utilizes a phenomenon in which singlet excitons are generated due to collision of two triplet excitons, and it is thought that the internal quantum efficiency can be theoretically raised to 40%. However, since the efficiency is lower compared to phosphorescent organic EL devices, further improvement in efficiency and low voltage characteristics are required.

In addition, patent Literature 2 discloses an organic EL device utilizing a TADF (Thermally Activated Delayed Fluorescence) mechanism. The TADF mechanism utilizes a phenomenon in which reverse intersystem crossing from triplet excitons to singlet excitons is generated in a material having a small energy difference between a singlet level and a triplet level, and it is thought that the internal quantum efficiency can be theoretically raised to 100%.

However, all the mechanisms have room for advancement in terms of both efficiency and life, and are additionally required to be improved also in terms of reduction in driving voltage.

Meanwhile, Patent Literatures 3 and 4 disclose use of an indolocarbazole compound with a substituted fused heterocycle, as a host material. Patent Literatures 5 and 6 disclose use of an indolocarbazole compound as a mixed host material. In addition, Patent Literature 7 discloses an organic EL device using a mixed host material of a compound of a nitrogen-containing 6-membered ring with a substituted fused heterocycle, and a carbazole compound. Moreover, Patent Literature 8 discloses an organic EL device using a mixed host material of an indolocarbazole compound of a nitrogen-containing 6-membered ring with a substituted fused heterocycle, and a specific indolocarbazole compound with a substituted aryl group. Furthermore, Patent Literatures 9 and 10 disclose an organic EL device using a mixed host material of indolocarbazole compounds.

However, none of these organic EL devices can be said to have sufficient properties, and further improvements particularly in efficiency and voltage are desired.

### Citation List

### Patent Literature

Patent Literature 1: WO2010/134350
Patent Literature 2: WO2011/070963
Patent Literature 3: WO2013/137001
Patent Literature 4: WO2013/122402
Patent Literature 5: WO2016/023608
Patent Literature 6: WO2018/198844
Patent Literature 7: Japanese Patent Laid-Open No. 2014-157947
Patent Literature 8: WO2018236092
Patent Literature 9: WO2016042997
Patent Literature 10: WO2010098246

### Summary of Invention

### Technical Problem

In order to apply organic EL devices to display devices such as flat panel displays, it is necessary to improve the luminous efficiency of devices and at the same time, to sufficiently secure the stability during driving. In view of the above circumstances, an object of the present invention is to provide a practically useful organic EL device realizing a high efficiency and low voltage characteristics.

### Solution to Problem

As a result of intensive studies, the present inventors have found that the above problem can be solved by an organic EL device using a specific mixed host material in a light-emitting layer, and have completed the present invention.

In other words, the present invention is an organic EL device having a plurality of organic layers between an anode and a cathode opposed to each other, wherein the organic layers have at least one light-emitting layer, the light-emitting layer comprises two host materials different from each other, and a dopant material, and one of the host materials is a compound represented by the following general formula (1) and the other of the host materials is a compound represented by the following general formula (2).

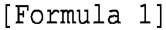

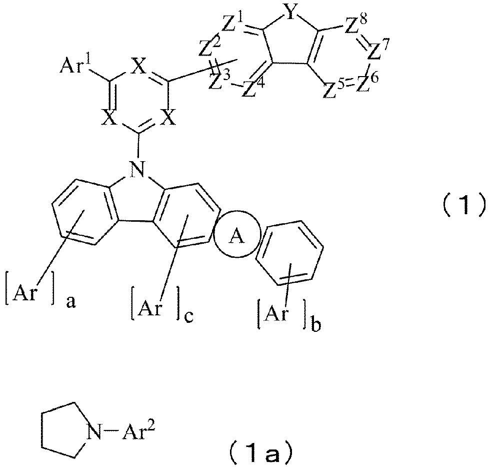

In the formula, a ring A is a heterocycle represented by formula (1a) and the ring A is fused at any position of an adjacent ring,
X represents N or C-Ar', wherein at least one X represents N.
Y represents O, S, N-Ar³, or C-Ar⁴Ar⁵.
Any one of Z¹ to Z⁴ represents a carbon atom binding to a 6-membered ring containing X, the others and Z⁵ to Z⁸ each independently represent C-Ar' or N, and Ar', when present in a plurality, may be the same or different.
Ar, Ar' and Ar¹ to Ar⁵ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other, provided that, when the group has a hydrogen atom, the hydrogen atom is optionally replaced with deuterium or halogen.
a and b represent the number of substitutions and each independently represent an integer of 1 to 4. c represents the number of substitutions and represents an integer of 1 to 2.

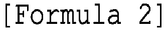

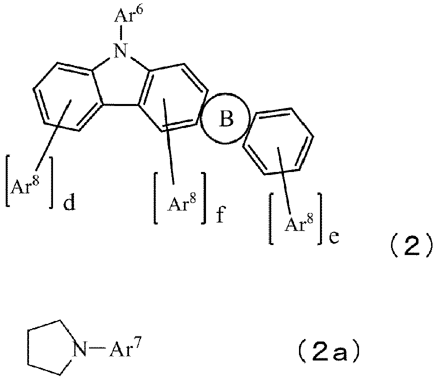

In the formula, a ring B is a heterocycle represented by formula (2a) and the ring B is fused at any position of an adjacent ring,
Ar⁶ and Ar⁷ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other, provided that, when the group has a hydrogen atom, the hydrogen atom is optionally replaced with deuterium or halogen.
d and e represent the number of substitutions and each represent an integer of 1 to 4. f represents the number of substitutions and represents an integer of 1 to 2.
Each Ar⁸ independently represents hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other.

In the general formula (1), any of Z¹ or Z² is preferably linked to a 6-membered ring containing X.

In the general formula (1), Y preferably represents O or S.

The general formula (1) is preferably represented by any of the following general formulas (4) to (8).

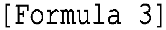

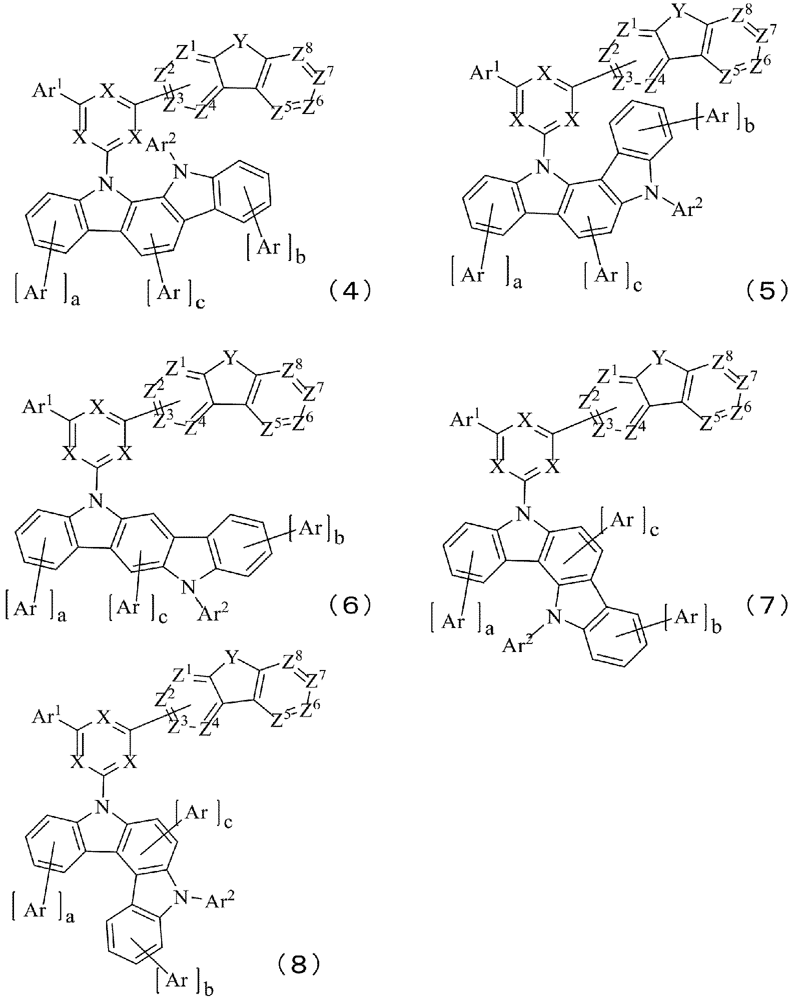

In the formulas, X, Y, Ar, Ar¹, Ar², Z¹ to Z⁸, a, b, and c have the same meaning as in the general formula (1).

In the general formulas (4) to (8), Z² is preferably linked to a 6-membered ring containing X.

In the general formulas (4) to (8), Ar¹ and Ar² each further preferably represent a substituted or unsubstituted aromatic hydrocarbon group having 6 to 10 carbon atoms, or a linked aromatic group in which two to five of these aromatic rings are linked to each other.

The general formula (2) is preferably represented by any of the following general formulas (9) to (13).

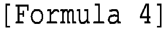

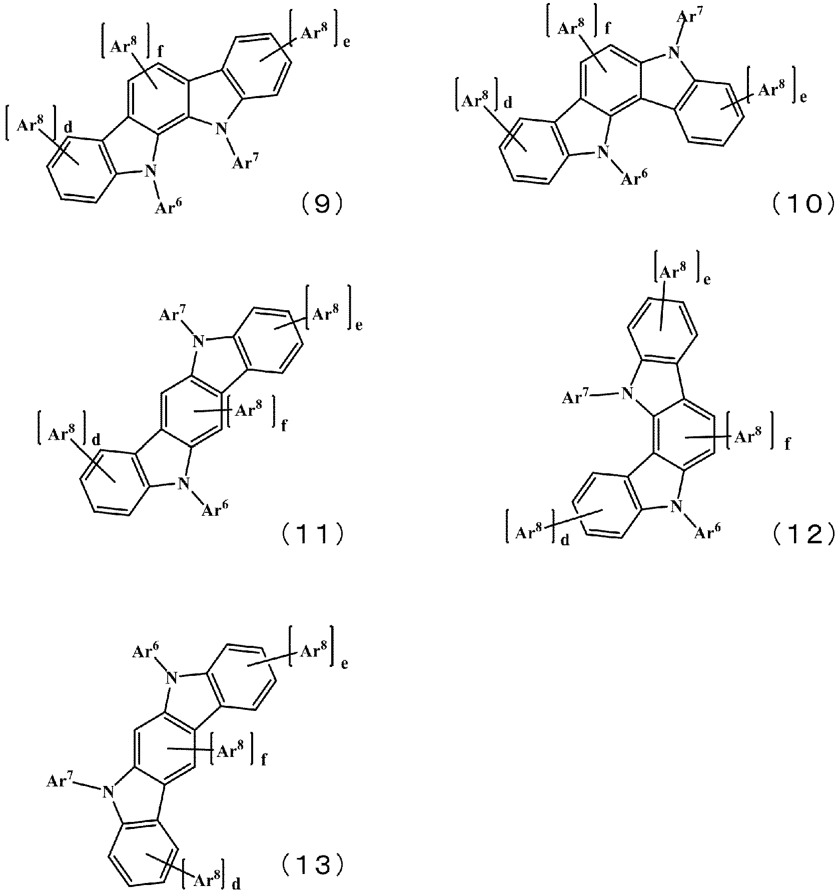

In the formulas, Ar⁸, d, e, f, Ar⁶ and Ar⁷ have the same meaning as in the formula (2).

In the general formulas (9) to (13), Ar⁶ and Ar⁷ each preferably represent a substituted or unsubstituted aromatic hydrocarbon group having 6 to 10 carbon atoms, a substituted or unsubstituted aromatic heterocyclic group having 3 to 17 carbon atoms, or a linked aromatic group in which two to five of these aromatic rings are linked to each other, and at least one of Ar⁶ and Ar⁷ preferably contains one or more aromatic heterocycles having 6 to 17 carbon atoms.

The dopant material is preferably a phosphorescent dopant material or a fluorescent dopant material emitting thermally activated delayed fluorescence.

Preferred aspects of the organic EL device of the present invention are here shown. In other words, the organic EL device according to the present invention has a mixed host material including two compounds, and also includes a light-emitting layer having a dopant material. The proportion of the compound represented by the general formula (1) based on the compound represented by the general formula (1) and the compound represented by the general formula (2) in total in the mixed host material is preferably 10 mass% or more and less than 70 mass%, more preferably 20 mass% or more and less than 60 mass%. The light-emitting dopant material is more preferably an organic metal complex containing at least one metal selected from the group consisting of ruthenium, rhodium, palladium, silver, rhenium, osmium, iridium, platinum and gold, or a fluorescent dopant material including thermally activated delayed fluorescence.

In addition, production of the organic EL device preferably has a step of mixing the compound represented by the general formula (1) and the compound represented by the general formula (2) to prepare a premixture, and vapor-depositing a host material containing the premixture to form a light-emitting layer.

In the above method for producing the organic EL device, it is suitable that a difference in 50% weight reduction temperatures of the compound represented by the general formula (1) and the compound represented by the general formula (2) is within 20°C.

For improvement of device characteristics, a higher durability of a material used for an organic layer against charges is needed, and particularly, in the light-emitting layer, it is important to reduce leakage of excitons and charges to surrounding layers. For leakage reduction of charges/excitons, it is effective to improve localization of a light emission area in the light-emitting layer. For this purpose, it is necessary to control amounts of both charges (electrons/holes) injected into the light-emitting layer or amounts of both charges transported in the light-emitting layer within a preferred range.

Both the compound represented by the general formula (1) and the compound represented by the general formula (2) used in the present invention each have an indolocarbazole structure. An indolocarbazole structure is known to have a high durability against charges. It is considered that the compound represented by the general formula (1), which has an indolocarbazole structure with a substituted nitrogen-containing 6-membered ring to which a fused aromatic group is linked, thus can allow for production of an organic EL device which is enhanced in injection transport properties of charges, particularly, electrons and which is stably driven even at a low voltage. It is considered that an indolocarbazole compound represented by the general formula (2) is mixed and thus an organic EL device can be produced which is enhanced in injection transport properties of charges, particularly, holes and which is stably driven even at a low voltage, and changing of the binding form of an indolocarbazole ring or the kind or number of substituents to a skeleton thereof allows a high-level control of charge injection transport properties, and thus it is presumed that a combination of the compound represented by the general formula (1) and the compound represented by the general formula (2) can control amounts of both charges injected into an organic layer within a preferable range and therefore localization of a light emission area in the light-emitting layer can be improved. It is expected that, in particular, in the case of a delayed fluorescence-emitting EL device or a phosphorescent EL device, they have a lowest excited triplet energy sufficiently high to confine the excitation energy generated in the light-emitting layer and therefore, an organic EL device having no outflow of energy from the inside of the light-emitting layer and having high efficiency and extended life at a low voltage can be produced.

### Advantageous Effects of Invention

Accordingly, the present invention can allow an organic EL device to be enhanced in characteristics, and in particular can allow for improvements in luminous efficiency and voltage applied, as compared with conventional one.

### Brief Description of Drawing

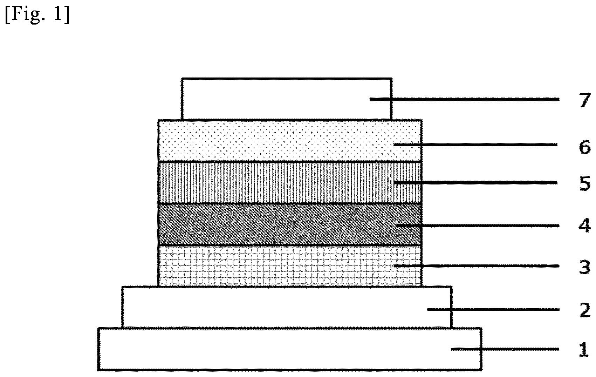

[Figure 1] Figure 1 is a schematic cross-sectional view showing one example of an organic EL device.

### Description of Embodiments

An organic EL device of the present invention has a structure wherein an anode, an organic layer and a cathode are laminated, wherein at least one layer of the organic layer includes a light-emitting layer formed by using a predetermined material for an organic EL device. In other words, the organic EL device has an organic layer composed of a plurality of layers between the anode and the cathode opposed to each other; and at least one layer of the plurality of layers is a light-emitting layer, and there may be a plurality of light-emitting layers. Then, at least one of the light-emitting layers includes two host materials different from each other, and a dopant material, one of the host materials includes the above compound represented by the general formula (1) (hereinafter, referred to as "first host material") and the other of the host materials includes the above compound represented by the general formula (2) (hereinafter, referred to as "second host material"), and these host materials form a light-emitting layer as a vapor deposition layer containing a light-emitting dopant material.

In other words, the first host material contained in the light-emitting layer is selected from the compounds represented by the general formula (1), and the second host material contained therein is selected from the compounds represented by the general formula (2).

In the general formula (1), X represents N or C-Ar', wherein at least one X represents N, two or more X preferably represent N, all of X more preferably represent N.

a and b represent the number of substitutions and each independently represents an integer of 1 to 4, preferably 1 to 2, more preferably 1. Similarly, c represents the number of substitutions and represents an integer of 1 to 2, preferably 1.

Any one of Z¹ to Z⁴ represents a carbon atom binding to a 6-membered ring containing X, and the others and Z⁵ to Z⁸ each independently represent C-Ar' or N, preferably C-Ar'. Ar', when present in a plurality, may be the same or different.

In the general formula (1), Ar, Ar' and Ar¹ to Ar⁵ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other.

In particular, Ar¹ and Ar² each more preferably represent a substituted or unsubstituted aromatic hydrocarbon group having 6 to 10 carbon atoms, or a linked aromatic group in which two to five of these aromatic rings are linked to each other.

Ar, Ar' and Ar³ to Ar⁵ each preferably represent hydrogen, deuterium, an alkyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, more preferably represent hydrogen, deuterium, a substituted or unsubstituted aromatic hydrocarbon group having 6 to 18 carbon atoms, a substituted or unsubstituted aromatic heterocyclic group having 3 to 12 carbon atoms, or a substituted or unsubstituted linked aromatic group in which two to five of these aromatic rings are linked to each other.

In the present specification, the linked aromatic group refers to a group in which aromatic rings of an aromatic hydrocarbon group and/or an aromatic heterocyclic group are linked by a single bond. Specifically, two to five of the aromatic rings of the substituted or unsubstituted aromatic hydrocarbon group having 6 to 30 carbon atoms are linked, or two to five of the aromatic rings of the substituted or unsubstituted aromatic heterocyclic group having 3 to 17 carbon atoms are linked, or two to five of the aromatic rings of the aromatic hydrocarbon group and the aromatic rings of the aromatic heterocyclic group are linked. These may be linked linearly or linked in a branched manner, and such aromatic rings may be the same or different.

In the case of Ar, Ar' and Ar¹ to Ar⁵ being halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, or an alkylsulfonyl group having 1 to 20 carbon atoms, specific examples thereof include alkyl groups such as methyl, ethyl, propyl, butyl, pentyl, cyclopentyl, hexyl, cyclohexyl, heptyl, octyl, nonyl, decyl, undecyl, dodecyl, tridecyl, tetradecyl, pentadecyl, hexadecyl, heptadecyl, octadecyl, nonadecyl, and icosyl, aralkyl groups such as phenylmethyl, phenylethyl, phenylicosyl, naphthylmethyl, anthranylmethyl, phenanthrenylmethyl, and pyrenylmethyl, alkenyl groups such as vinyl, propenyl, butenyl, pentenyl, decenyl, and icosenyl, alkynyl groups such as ethynyl, propargyl, butynyl, pentynyl, decynyl, and icosynyl, dialkylamino groups such as dimethylamino, ethylmethylamino, diethylamino, dipropylamino, dibutylamino, dipentynylamino, didecylamino, and diicosylamino, diarylamino groups such as diphenylamino, naphthylphenylamino, dinaphthylamino, dianthranylamino, diphenanthrenylamino, and dipyrenylamino, diaralkylamino groups such as diphenylmethylamino, diphenylethylamino, phenylmethylphenylethylamino, dinaphthylmethylamino, dianthranylmethylamino, and diphenanthrenylmethylamino, acyl groups such as acetyl, propionyl, butyryl, valeryl, and benzoyl, acyloxy groups such as acetyloxy, propionyloxy, butyryloxy, valeryloxy, and benzoyloxy, alkoxy groups such as methoxy, ethoxy, propoxy, butoxy, pentoxy, hexoxy, heptoxy, octoxy, nonyloxy, and decanyloxy, alkoxycarbonyl groups such as methoxycarbonyl, ethoxycarbonyl, propoxycarbonyl, butoxycarbonyl, and pentoxycarbonyl, alkoxycarbonyloxy groups such as methoxycarbonyloxy, ethoxycarbonyloxy, propoxycarbonyloxy, butoxycarbonyloxy, and pentoxycarbonyloxy, alkylsulfonyl groups such as methylsulfonyl, ethylsulfonyl, propylsulfonyl, butylsulfonyl, and pentylsulfonyl, a cyano group, a nitro group, a fluoro group, and a tosyl group. Preferred is methyl, ethyl, propyl, butyl, pentyl, cyclopentyl, hexyl, cyclohexyl, heptyl, octyl, nonyl, decyl, undecyl, or dodecyl.

In the case of unsubstituted Ar, Ar' and unsubstituted Ar³ to Ar⁵ being an aromatic hydrocarbon group, an aromatic heterocyclic group, or a linked aromatic group, specific examples thereof include an aromatic group generated by removing one H from benzene, naphthalene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, benzothiadiazole, dibenzofuran, dibenzothiophene, dibenzoselenophene, carbazole, or a compound constituted by linking of two to five of these. Preferred examples include an aromatic group generated by removing one H from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, benzothiadiazole, dibenzofuran, dibenzothiophene, dibenzoselenophene, carbazole, or a compound constituted by linking of two to five of these. More preferred examples include an aromatic group generated by removing one H from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, dibenzofuran, dibenzothiophene, carbazole, or a compound constituted by linking of two to five of these.

In the case of unsubstituted Ar¹ and Ar² each being an aromatic hydrocarbon group, an aromatic heterocyclic group, or a linked aromatic group, specific examples thereof are the same as those of unsubstituted Ar and unsubstituted Ar³ to Ar⁵, and preferred examples include an aromatic group generated by removing one H from benzene, naphthalene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, benzothiadiazole, dibenzofuran, dibenzothiophene, dibenzoselenophene, carbazole, or a compound constituted by linking of two to five of these. More preferred examples include an aromatic group generated by removing one H from benzene, naphthalene, or a compound constituted by linking of two to five of these.

In the present specification, the above unsubstituted aromatic hydrocarbon group or aromatic heterocyclic group may each have a substituent. In the case of the presence of a substituent, the substituent is a cyano group, an aliphatic hydrocarbon group having 1 to 10 carbon atoms, or a diarylamino group having 12 to 44 carbon atoms.

When the substituent is an aliphatic hydrocarbon group having 1 to 10 carbon atoms, it may be linear, branched, or cyclic.

The number of the substituents is 0 to 5 and is preferably 0 to 2. When the aromatic hydrocarbon group and the aromatic heterocyclic group have the substituent, the number of carbon atoms is calculated except for the number of carbon atoms in the substituent. However, the total number of carbon atoms, including the number of carbon atoms in the substituent, preferably satisfies the above range. Herein, when the group has a hydrogen atom, the hydrogen atom is optionally replaced with deuterium or halogen.

Specific examples of the substituent include cyano, methyl, ethyl, propyl, i-propyl, butyl, t-butyl, pentyl, cyclopentyl, hexyl, cyclohexyl, heptyl, octyl, nonyl, decyl, diphenylamino, naphthylphenylamino, dinaphthylamino, dianthranylamino, diphenanthrenylamino, and dipyrenylamino. Preferred examples include cyano, methyl, ethyl, propyl, butyl, pentyl, hexyl, heptyl, octyl, diphenylamino, naphthylphenylamino, or dinaphthylamino.

Specific examples of the compound represented by the general formula (1) are shown below, but not limited thereto.

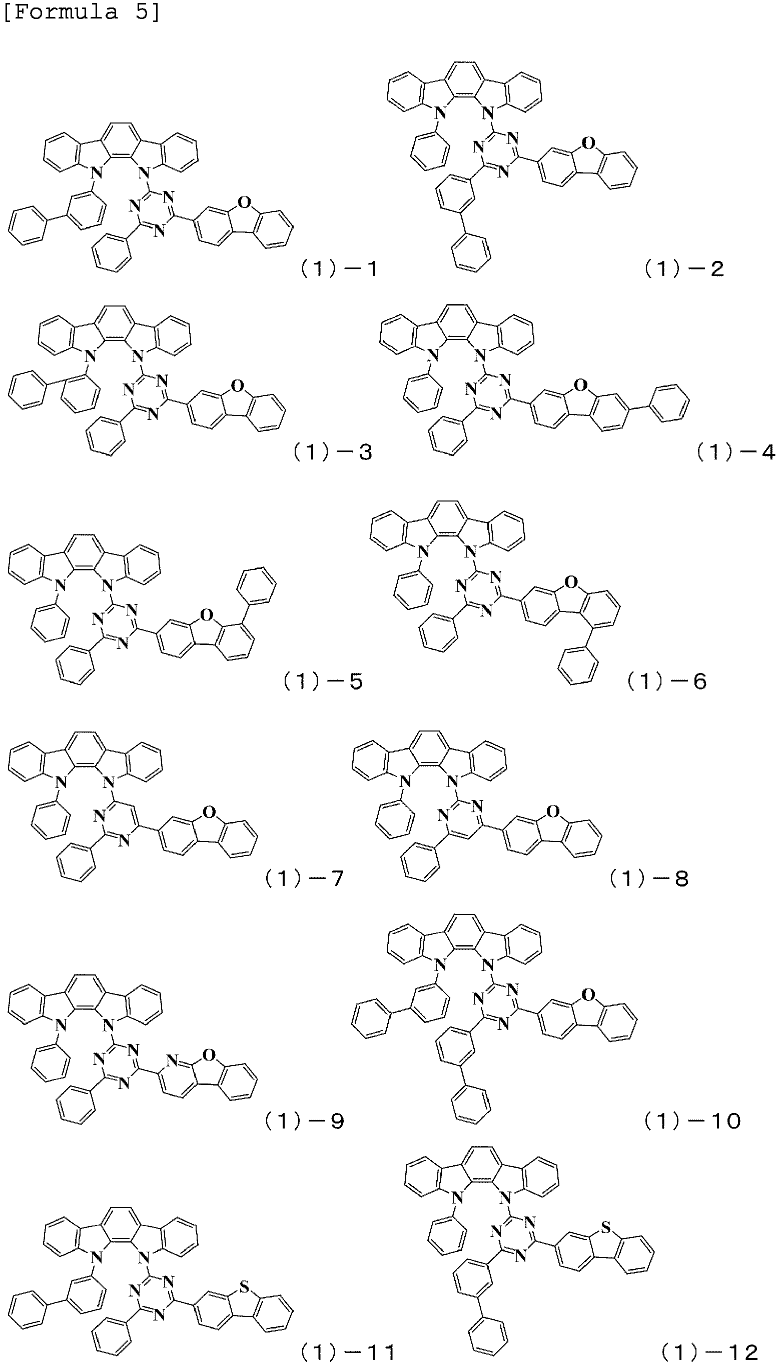

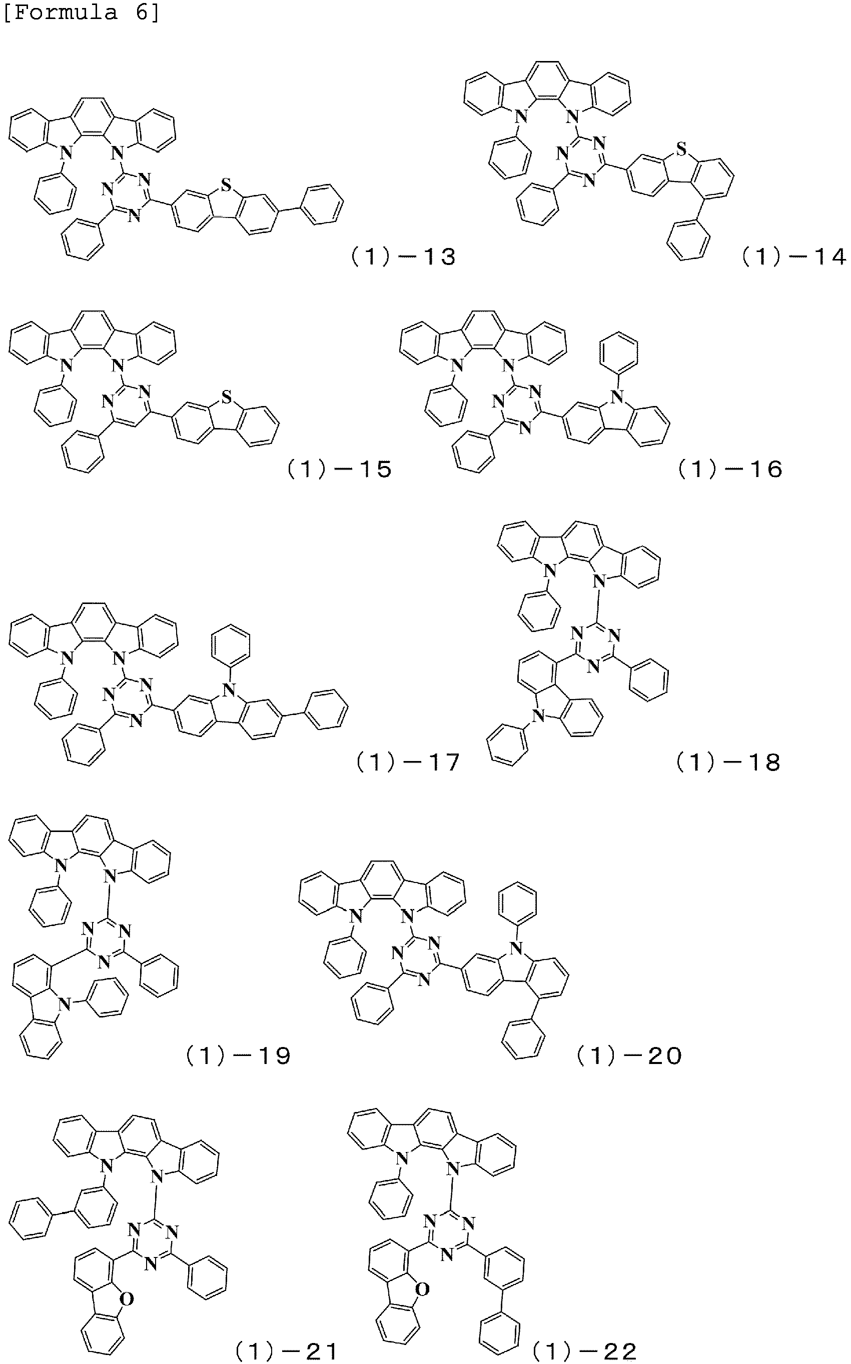

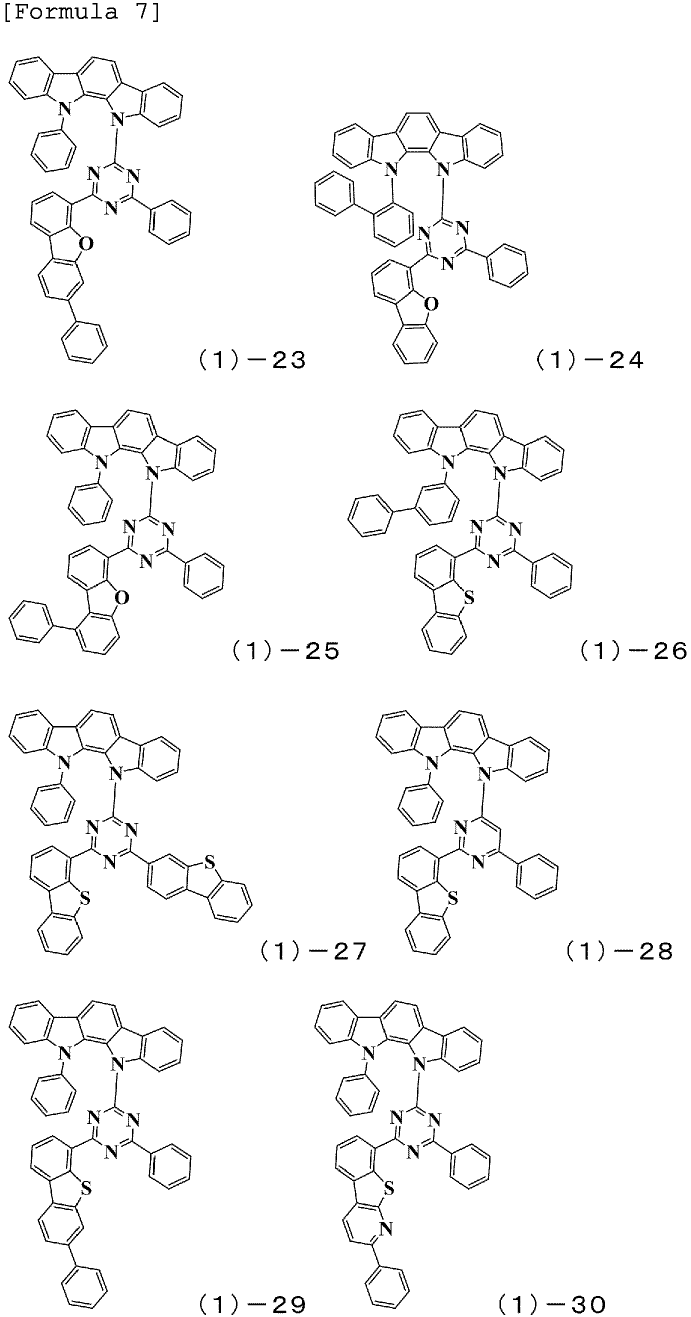

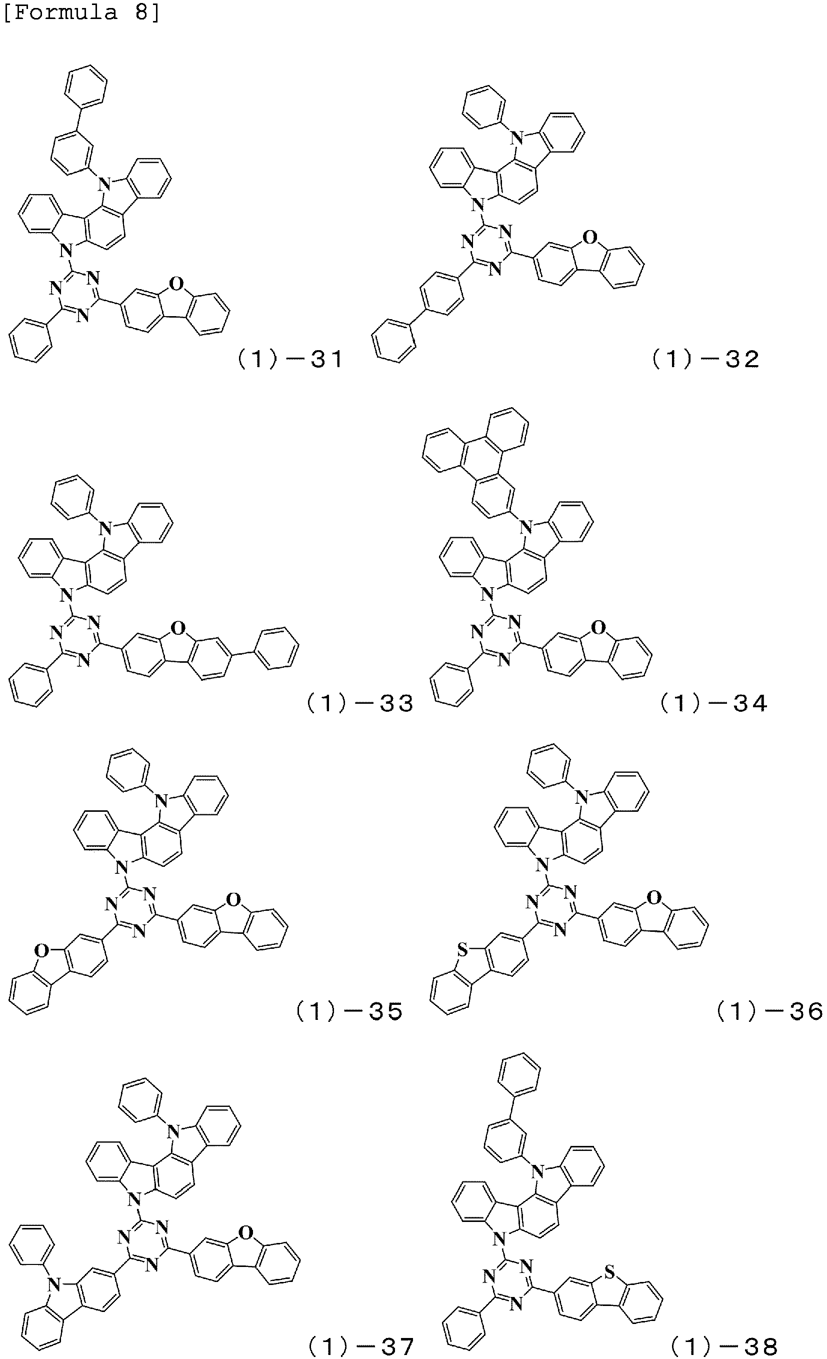

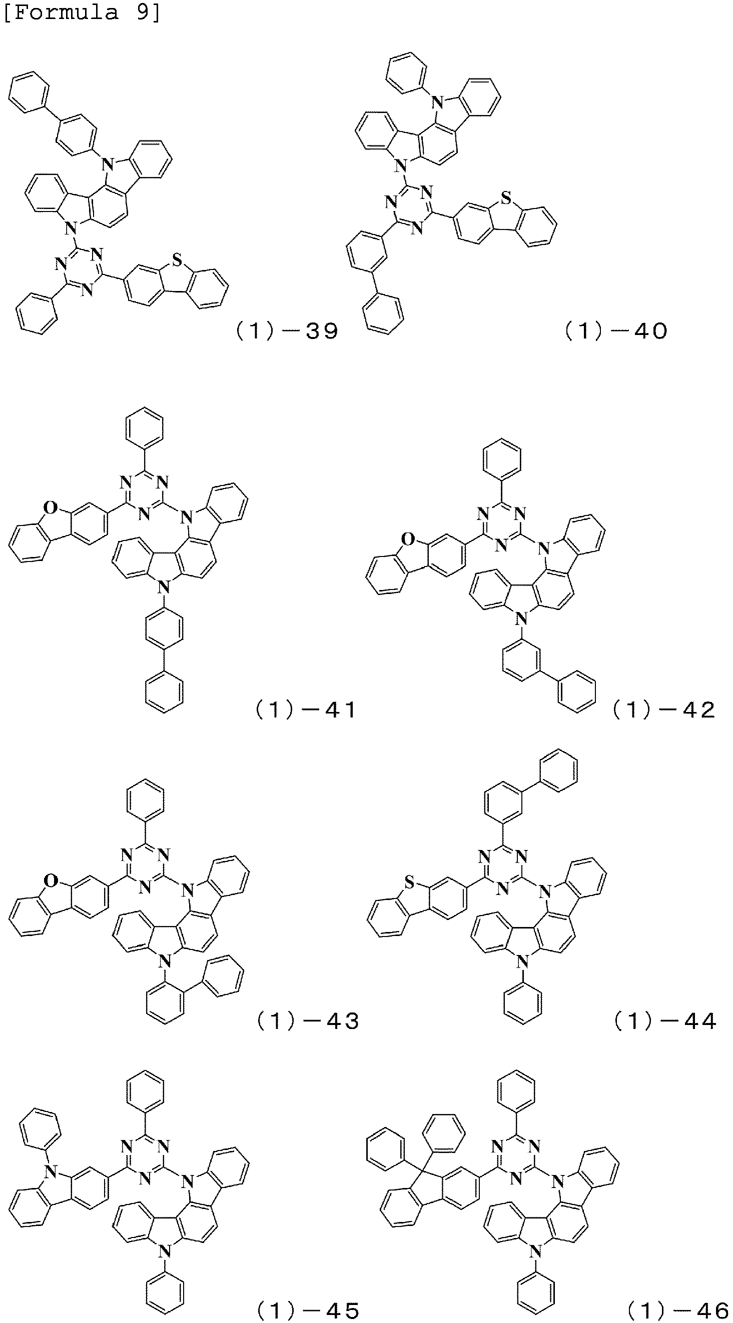

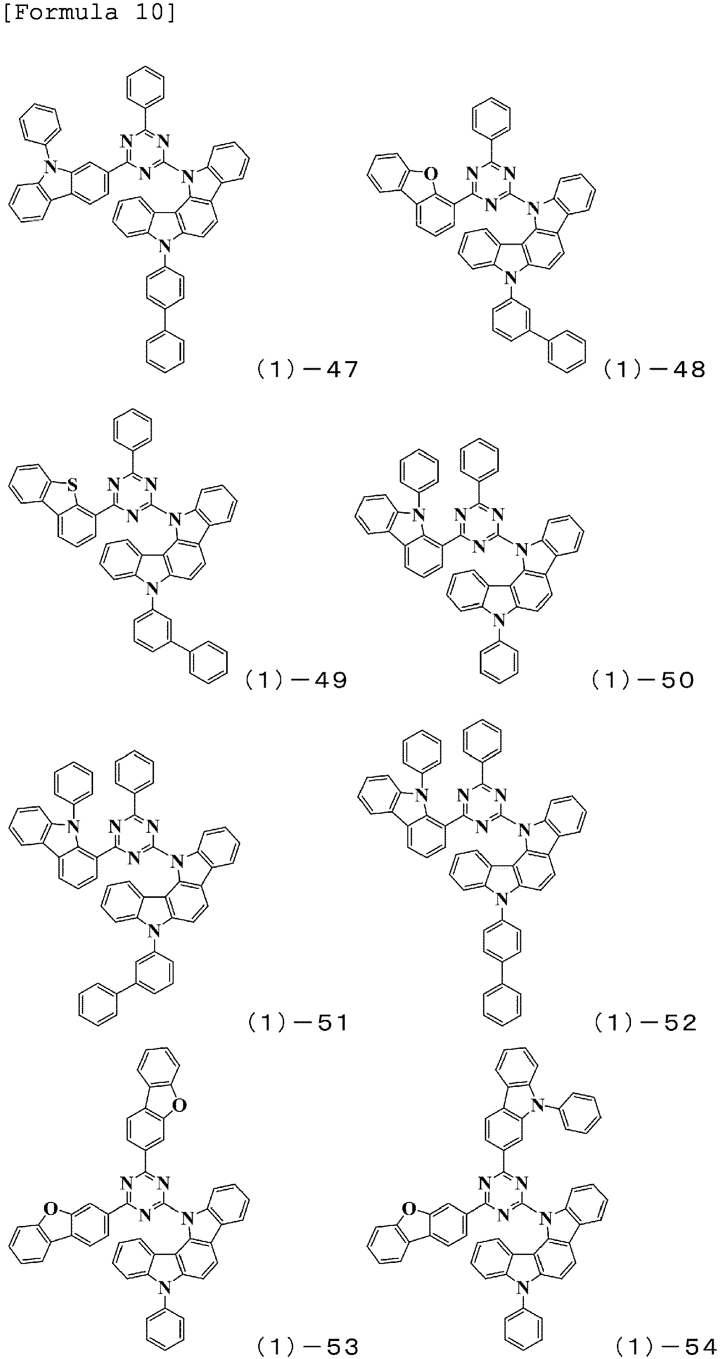

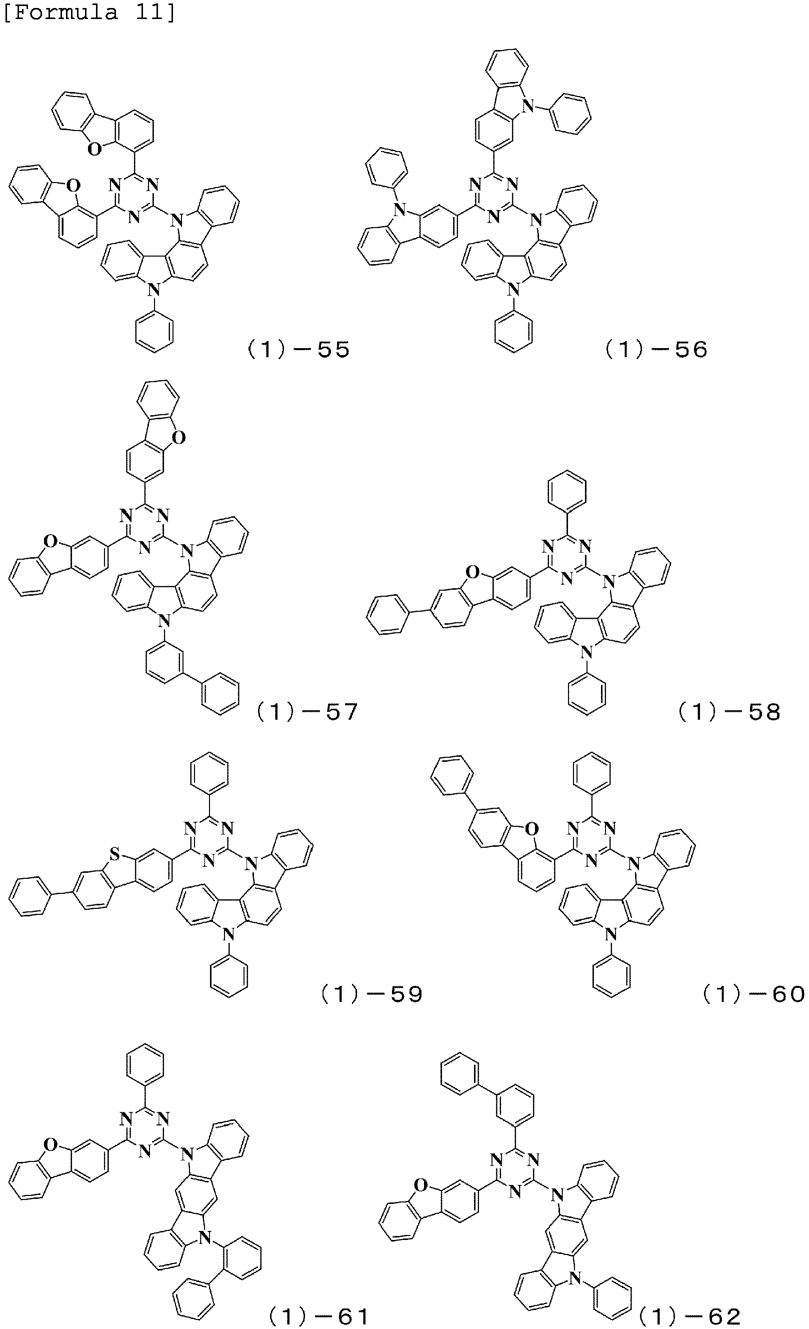

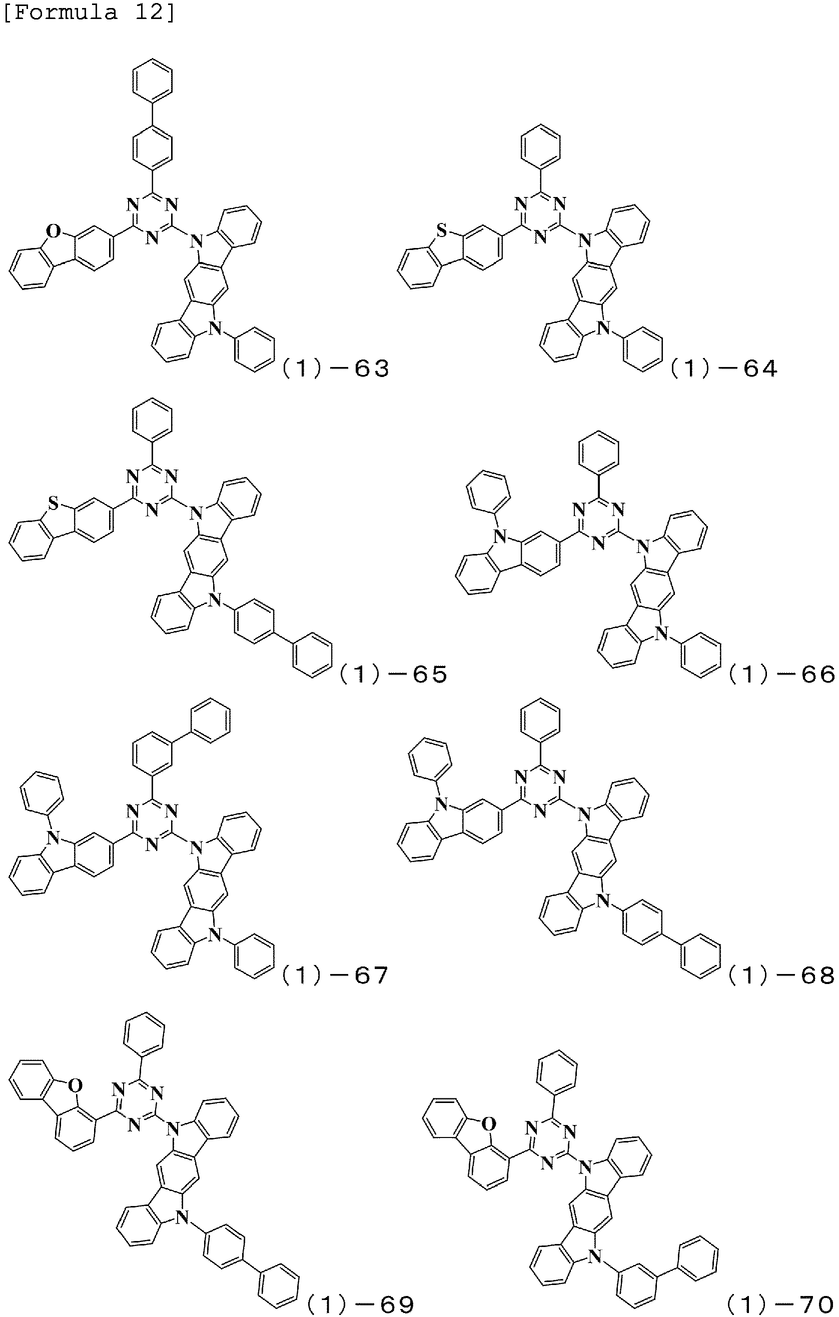

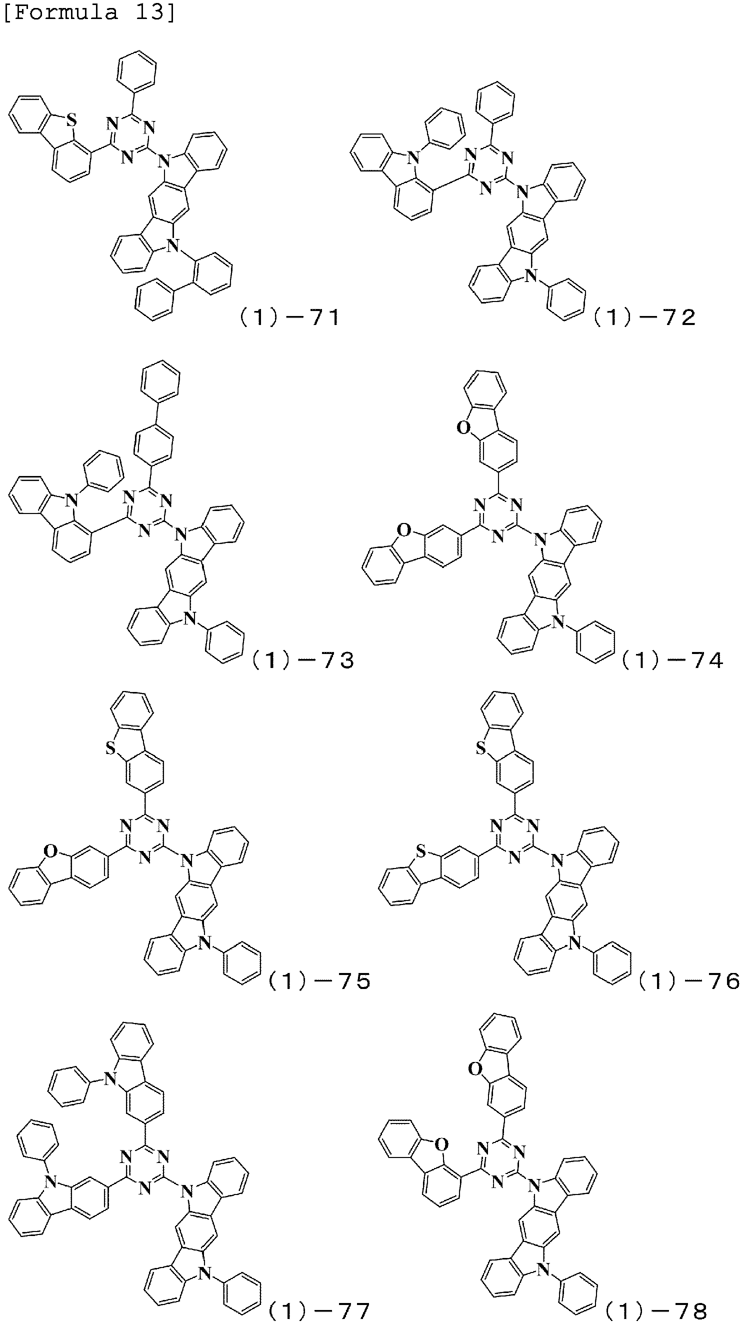

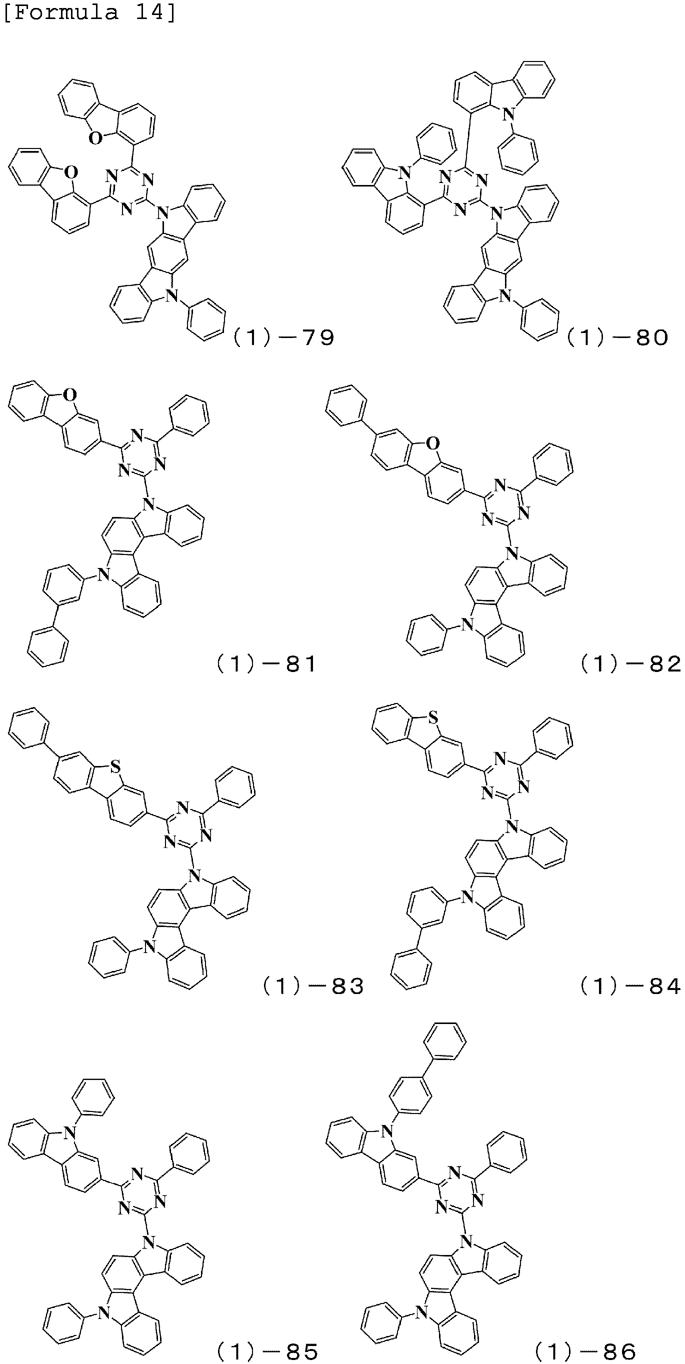

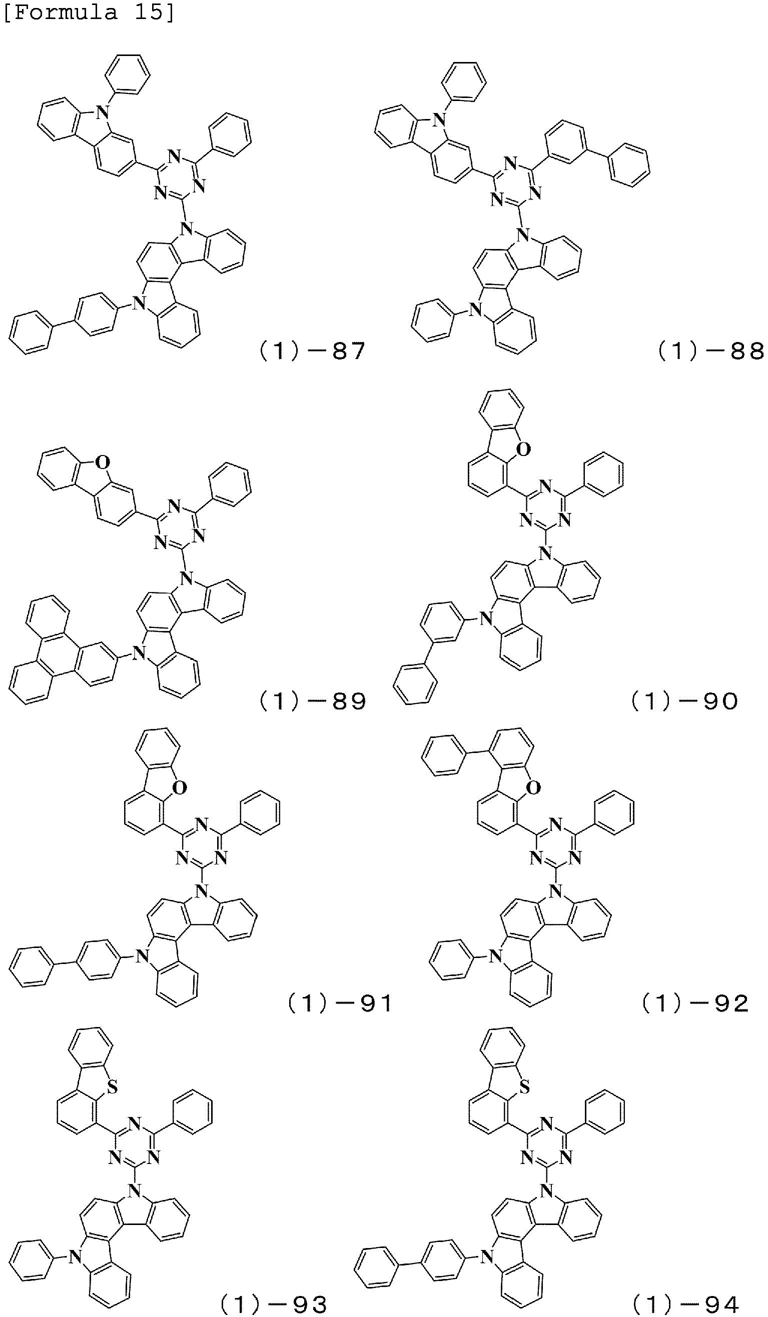

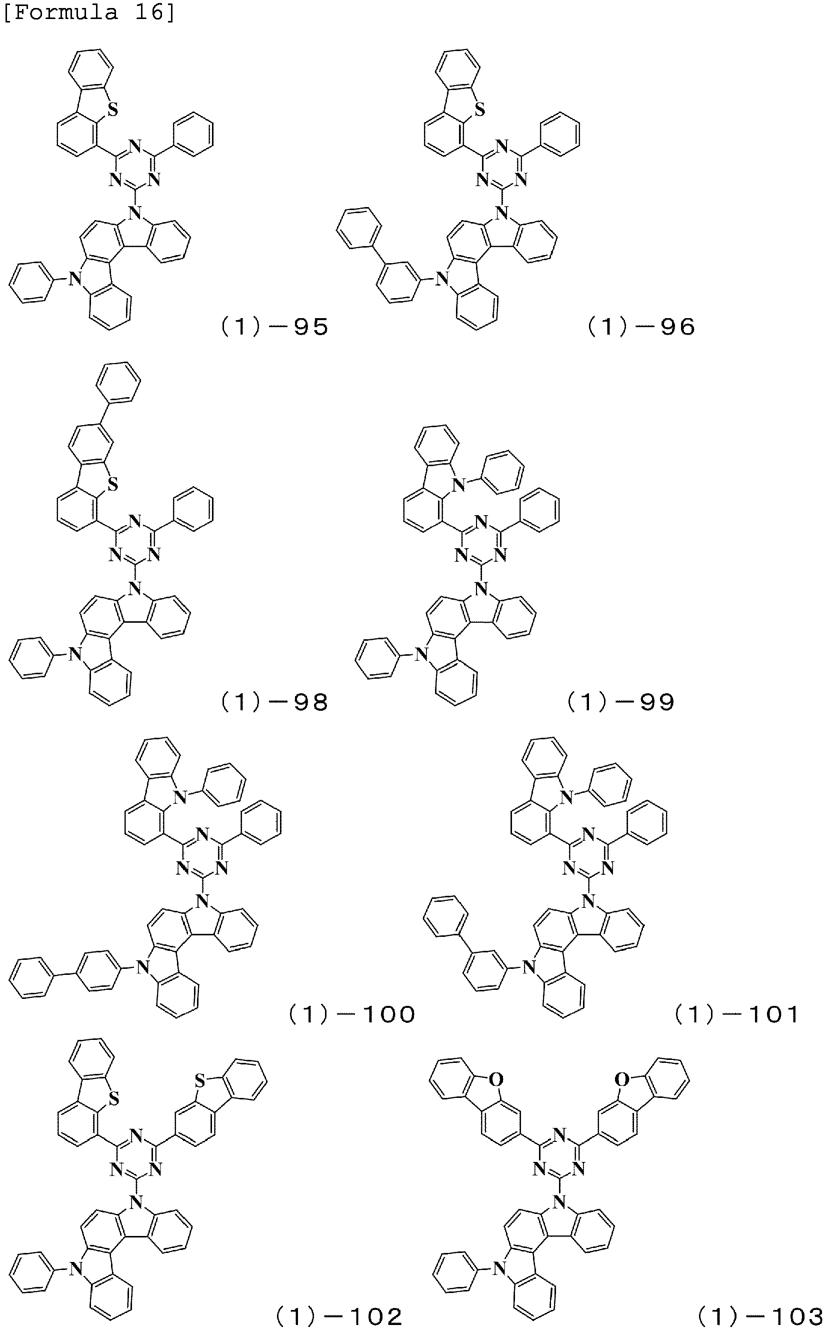

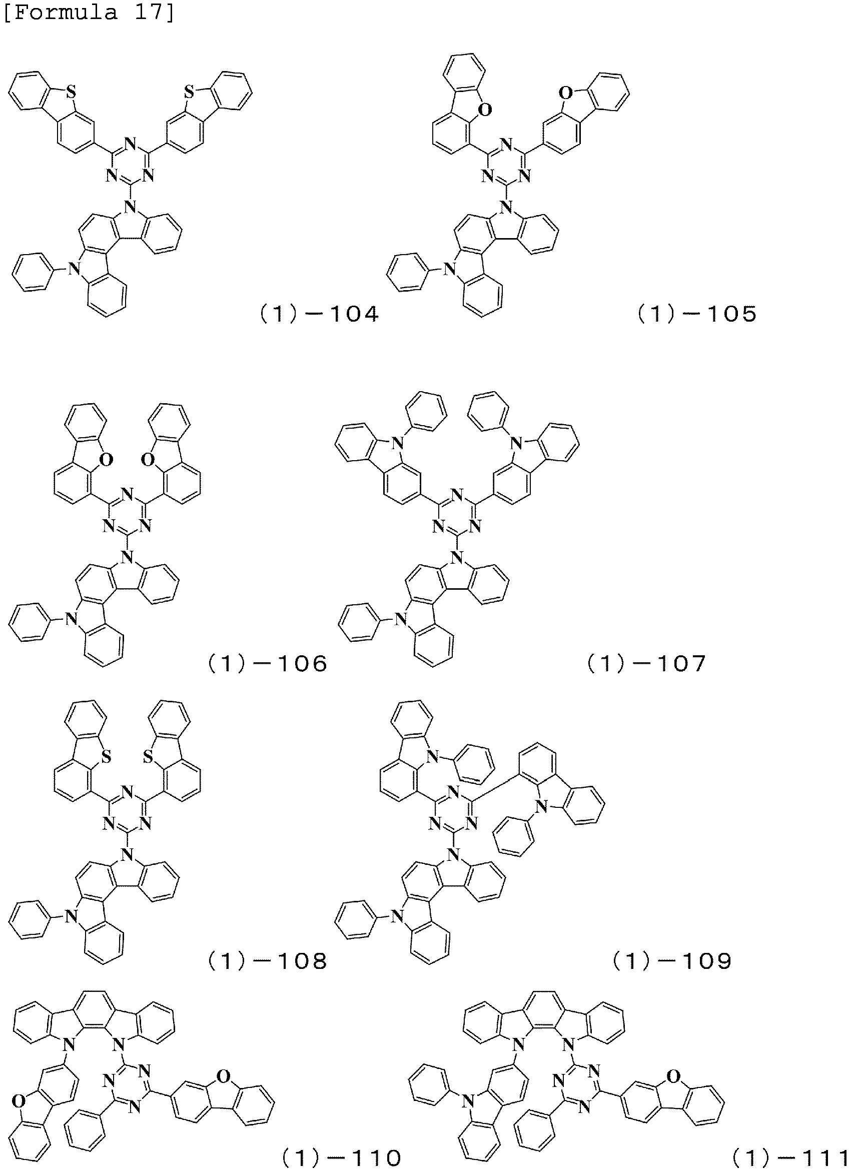

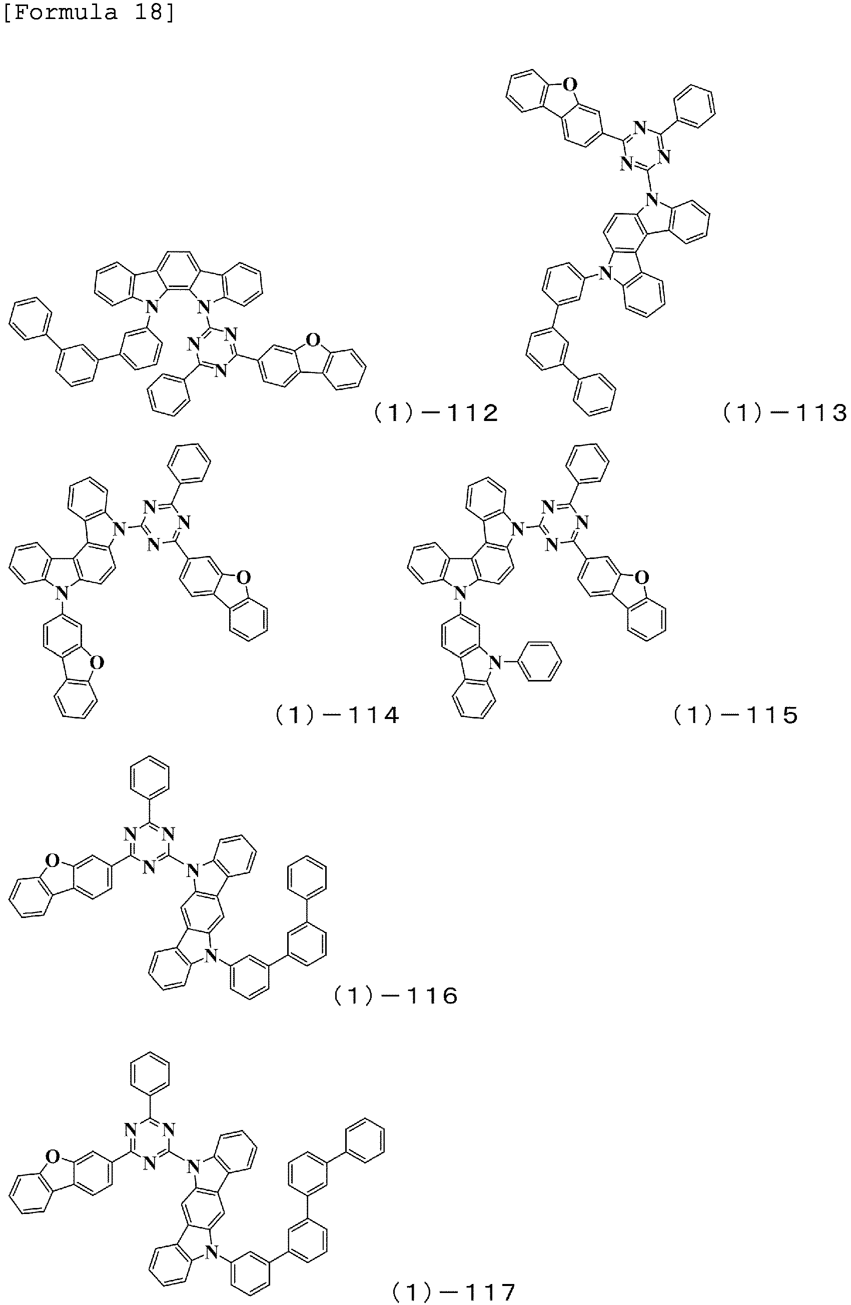

In the general formula (2), Ar⁶ and Ar⁷ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic hydrocarbon groups and aromatic heterocyclic groups are linked to each other, preferably represent a substituted or unsubstituted aromatic hydrocarbon group having 6 to 18 carbon atoms, a substituted or unsubstituted aromatic heterocyclic group having 6 to 17 carbon atoms, or a linked aromatic group in which two to five of these aromatic hydrocarbon groups and aromatic heterocyclic groups are linked to each other, and at least one of Ar⁶ and Ar⁷ more preferably contains one or more substituted or unsubstituted aromatic heterocycles having 6 to 17 carbon atoms.

In the case of unsubstituted Ar⁶ and Ar⁷ each being an aromatic hydrocarbon group, an aromatic heterocyclic group, or a linked aromatic group, specific examples thereof include an aromatic group generated by removing one H from benzene, naphthalene, pyridine, pyrimidine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, benzothiadiazole, dibenzofuran, dibenzothiophene, dibenzoselenophene, carbazole, or a compound constituted by linking of two to five of these. Preferred examples include an aromatic group generated by removing one H from benzene, naphthalene, triphenylene, pyridine, pyrimidine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzoisothiazole, benzothiadiazole, dibenzofuran, dibenzothiophene, dibenzoselenophene, carbazole, or a compound constituted by linking of two to five of these. More preferred examples include an aromatic group generated by removing one H from benzene, carbazole, or a compound constituted by linking of two to five of these.

d and e represent the number of substitutions and each represent an integer of 1 to 4, preferably 1 to 2, more preferably 1. Similarly, f represents the number of substitutions and represents an integer of 1 to 2, preferably 1.

Each Ar⁸ independently represents hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other.

Ar⁸ preferably represents hydrogen, deuterium, an alkyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, more preferably represents hydrogen, deuterium, a substituted or unsubstituted aromatic hydrocarbon group having 6 to 18 carbon atoms, a substituted or unsubstituted aromatic heterocyclic group having 3 to 12 carbon atoms, or a substituted or unsubstituted linked aromatic group in which two to five of these aromatic rings are linked to each other.

Specific examples of Ar⁸ are the same as those noted with respect to Ar.

In the case of Ar⁶ and Ar⁷ each being an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, or an alkylsulfonyl group having 1 to 20 carbon atoms, specific examples thereof are the same as in Ar, Ar' and Ar¹ to Ar⁵. In the present invention, a light-emitting layer including two host materials is formed, and thus the compound represented by the general formula (1) and the compound represented by the general formula (2) are different from each other. Therefore, Ar⁶ and Ar⁷ in the general formula (2) are not represented by the following formula (3), for the purpose of avoidance of overlapping of both the compounds.

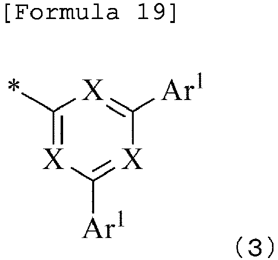

In the formula, ^{∗} represents a position for linking with the general formula (2), and X and Ar¹ have the same meaning as in the general formula (1).

Specific examples of the compound represented by the general formula (2) are shown below, but not limited thereto.

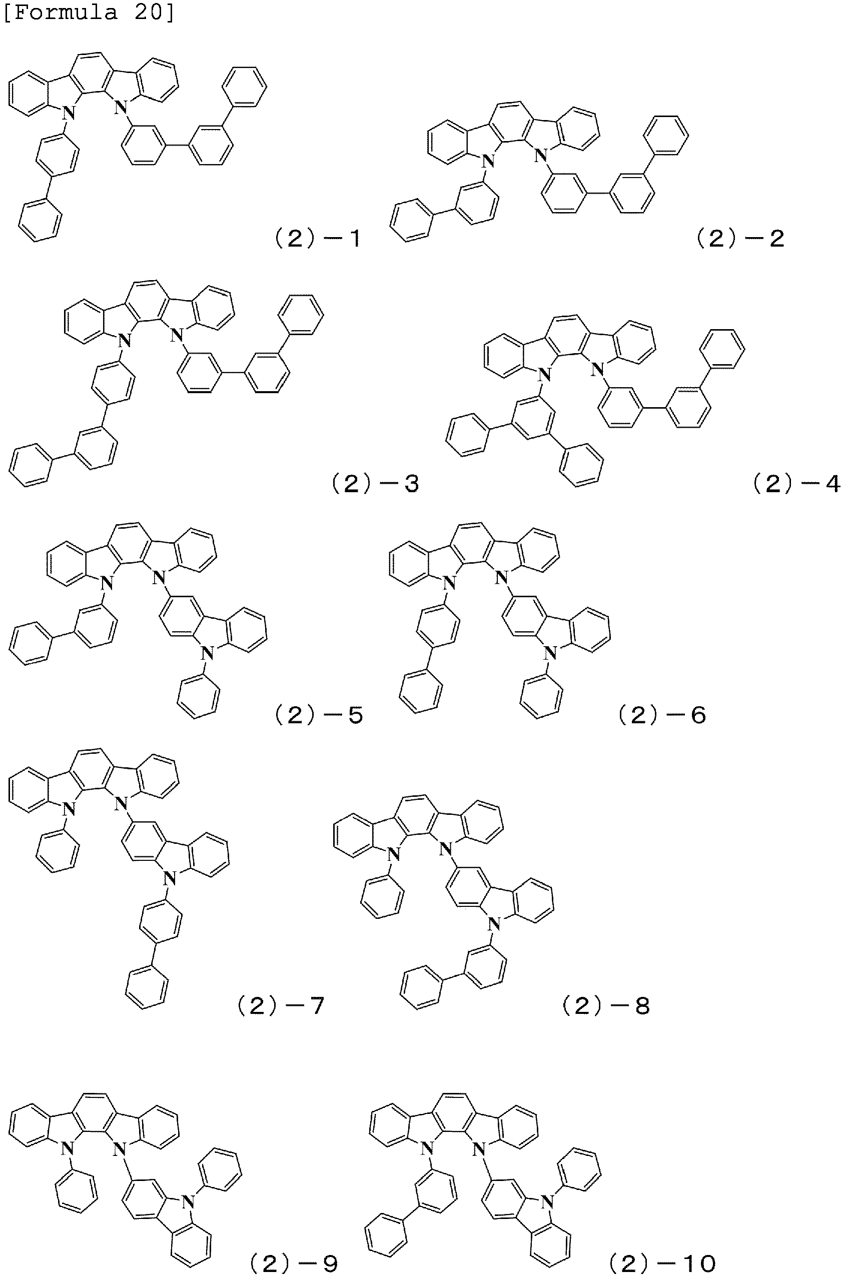

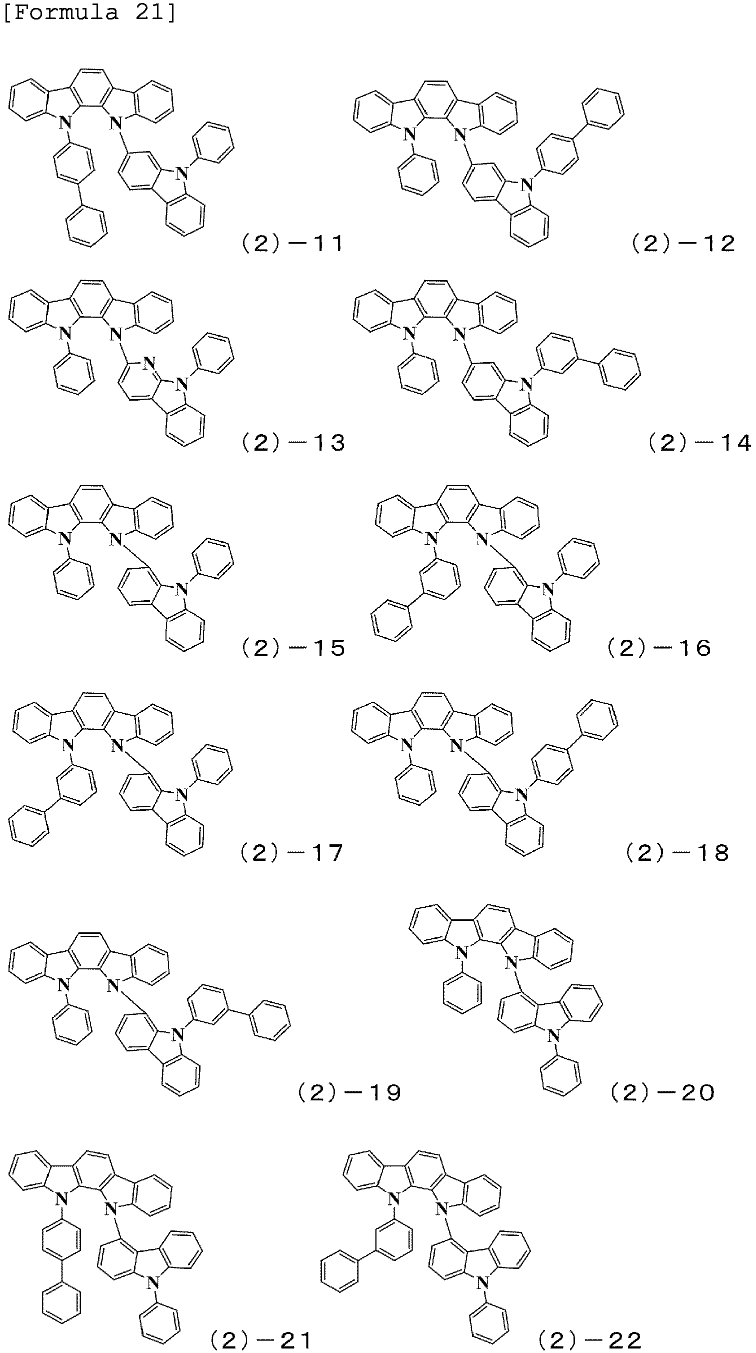

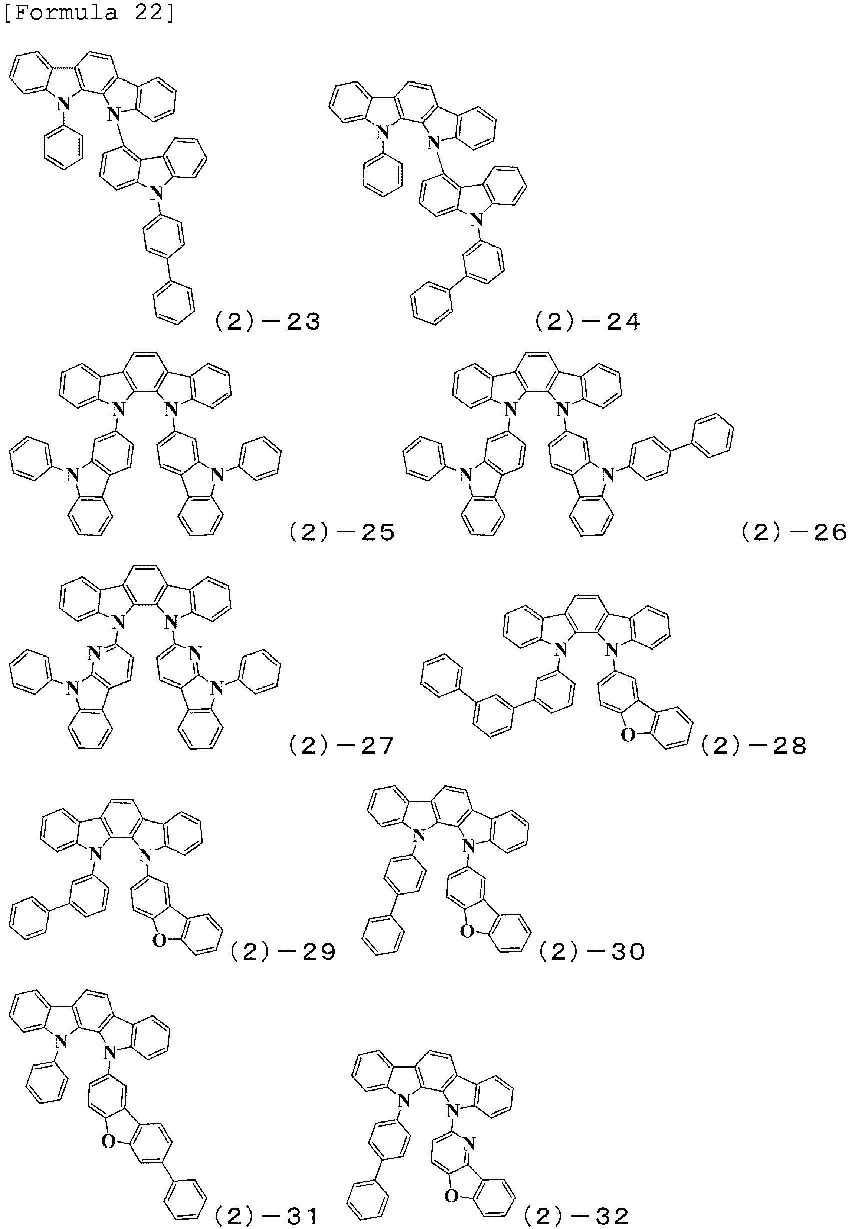

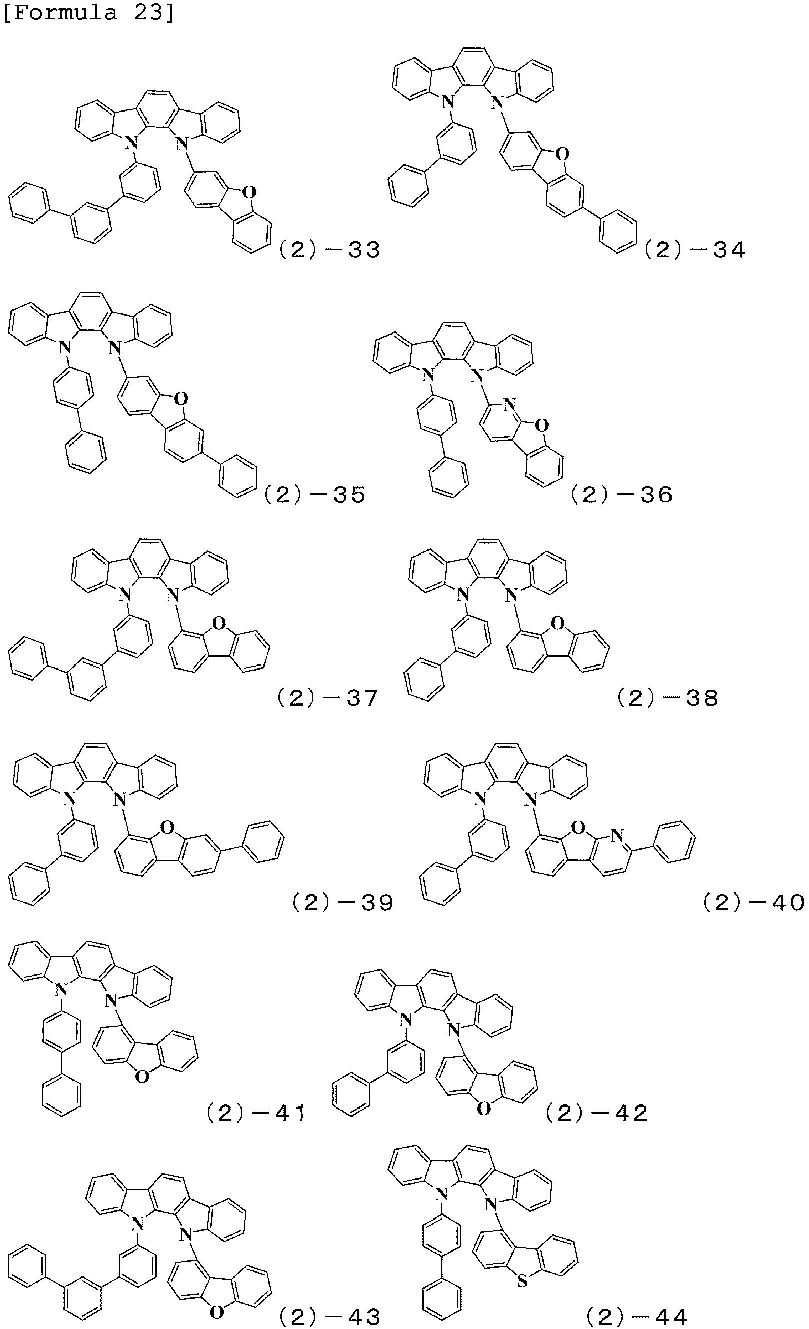

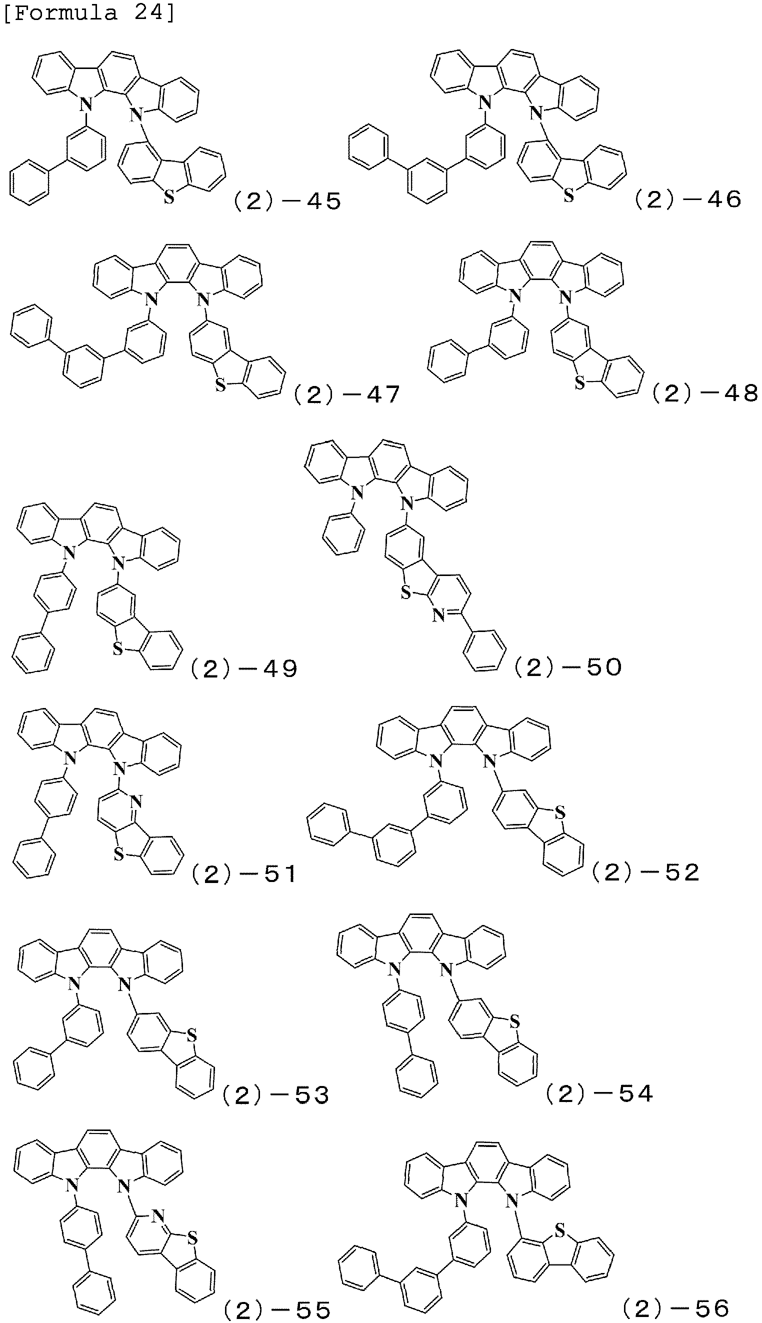

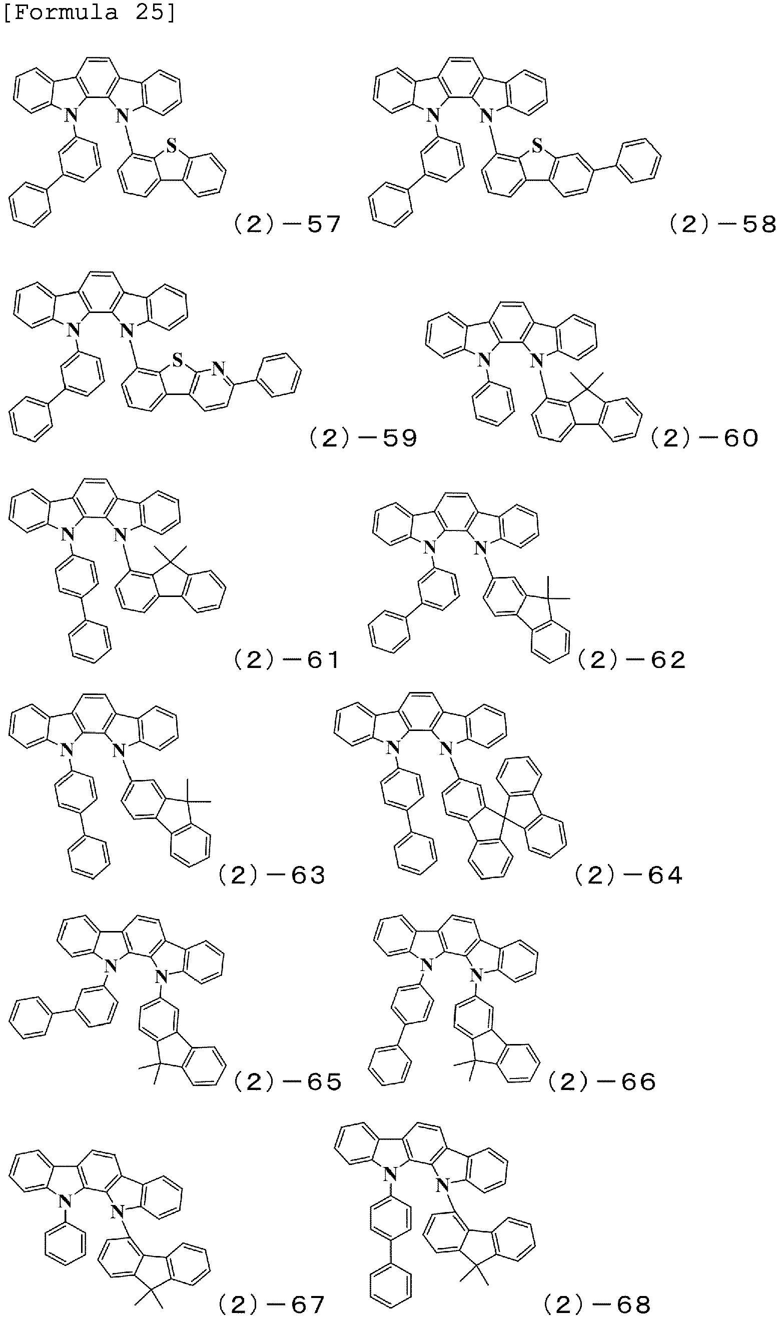

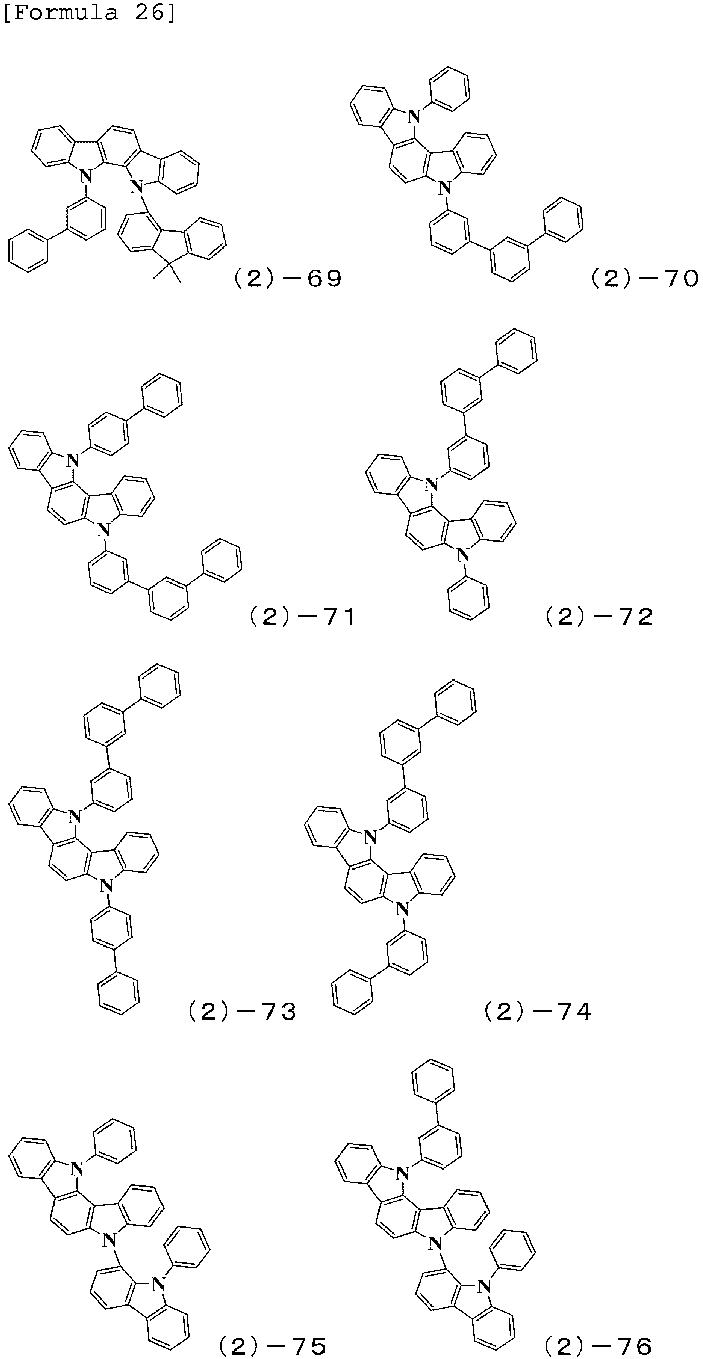

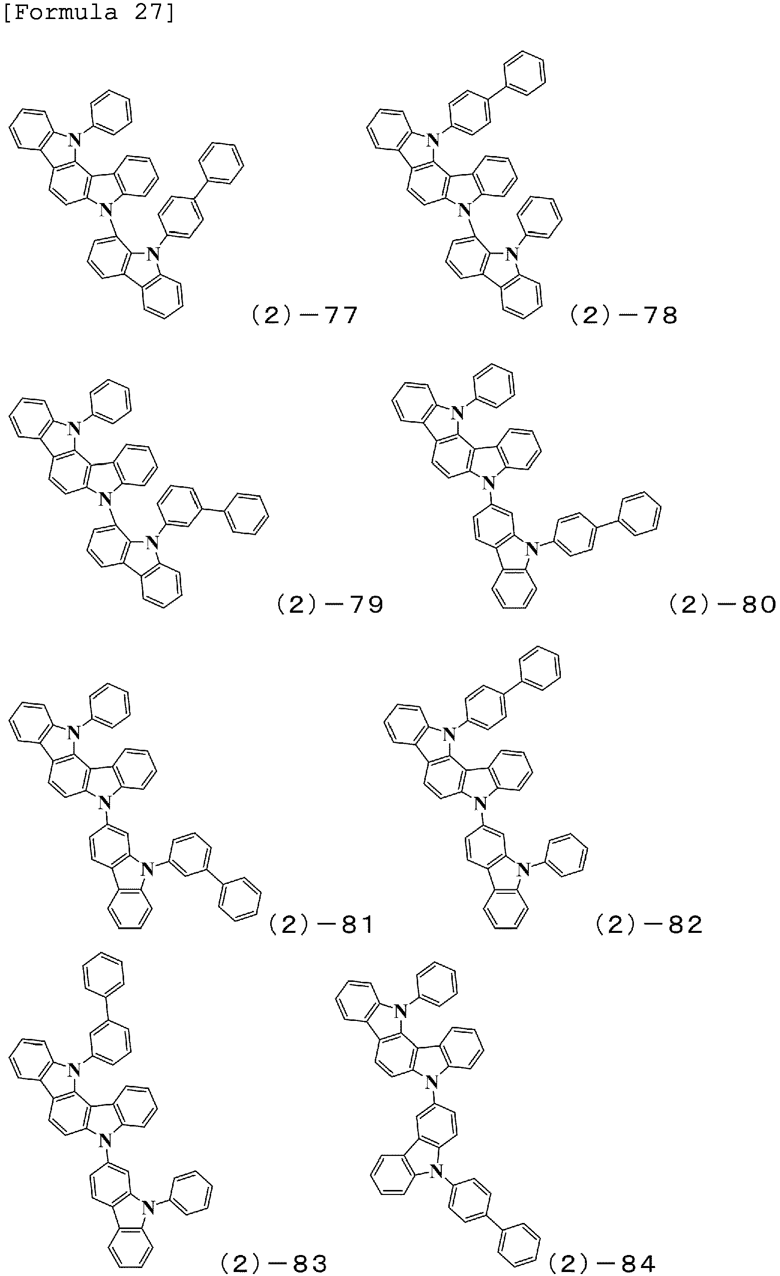

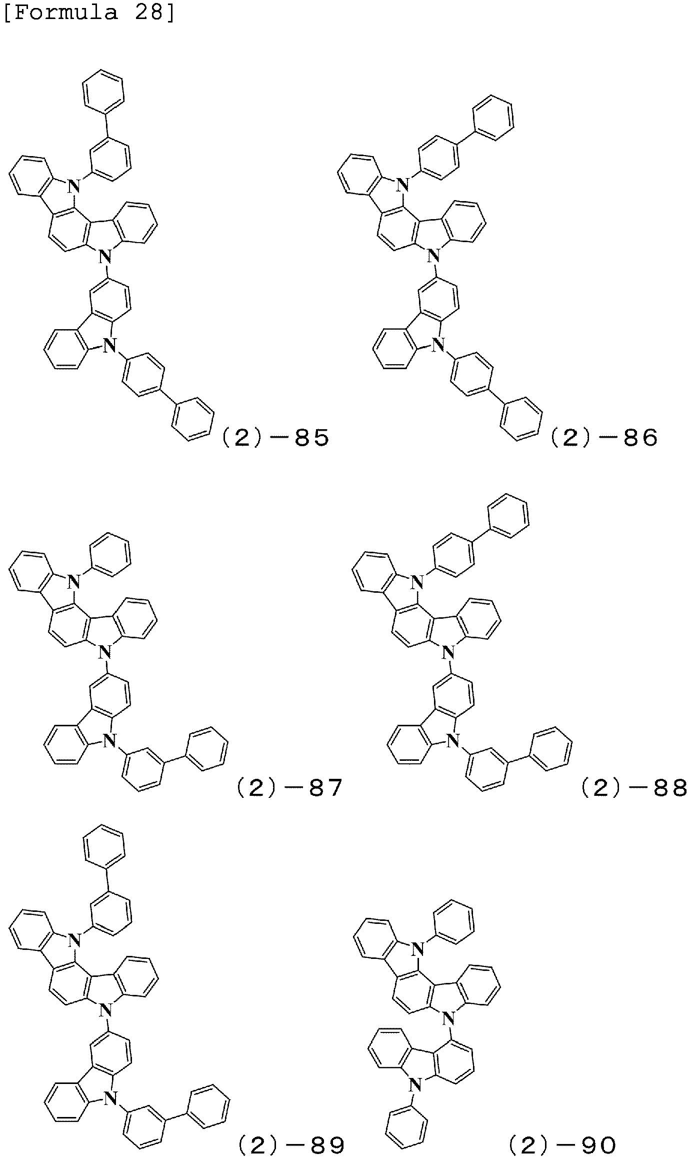

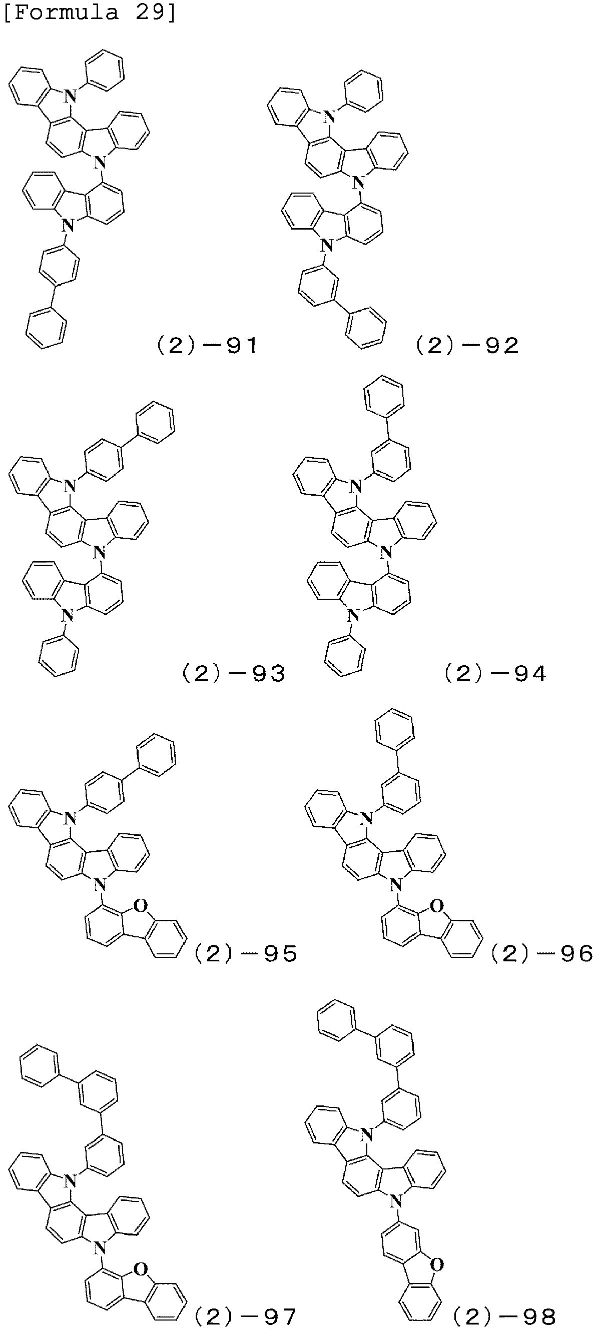

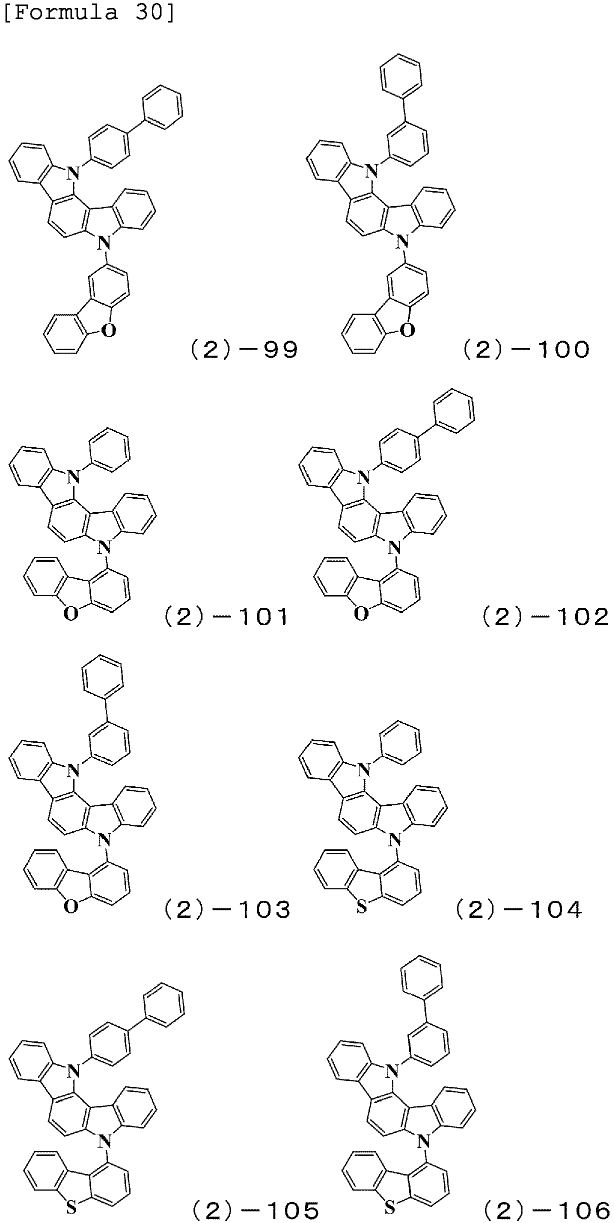

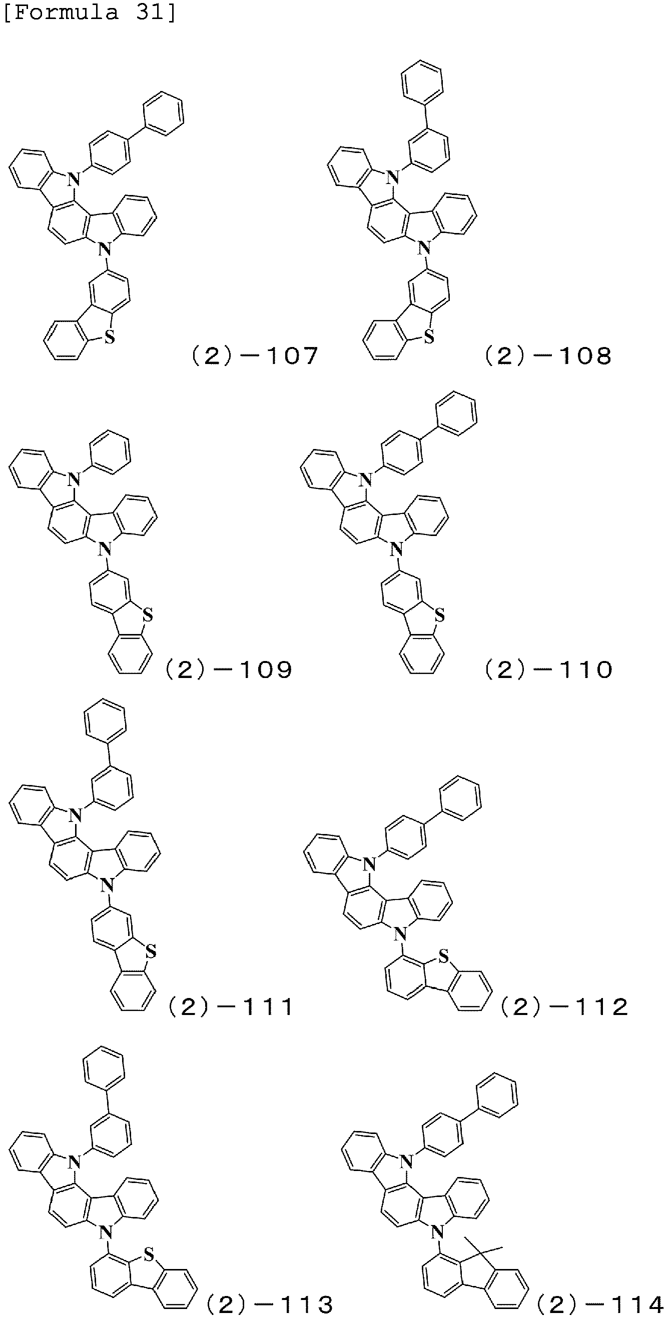

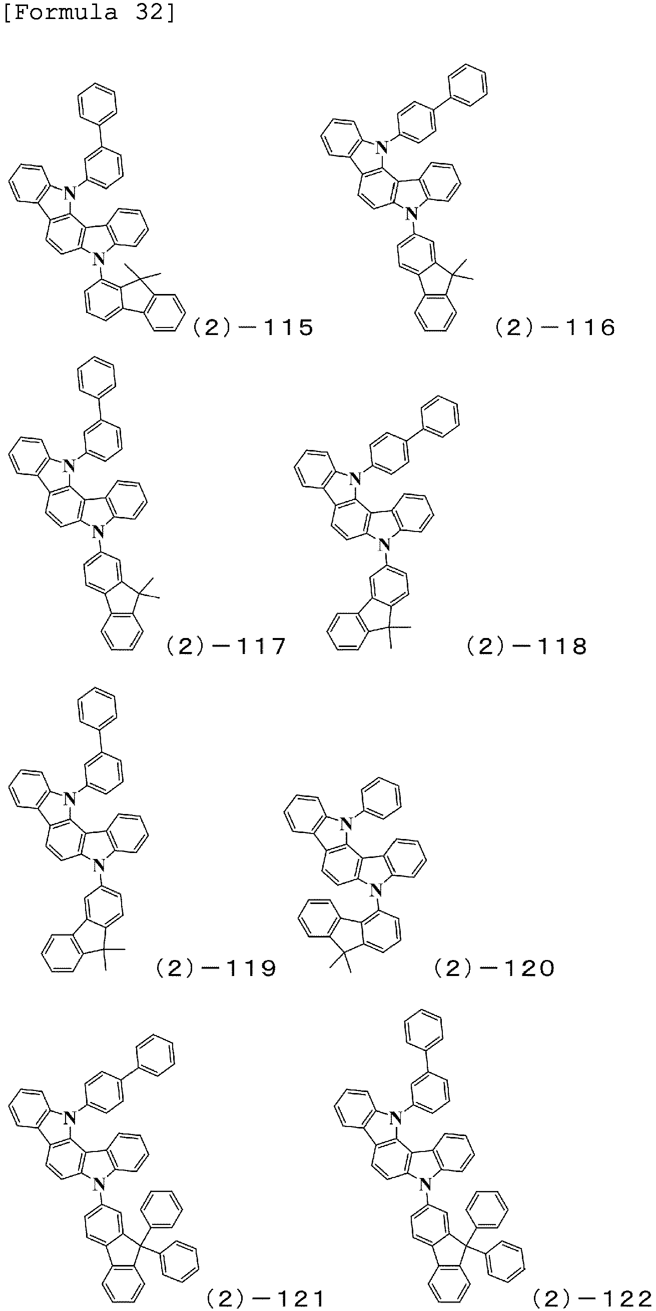

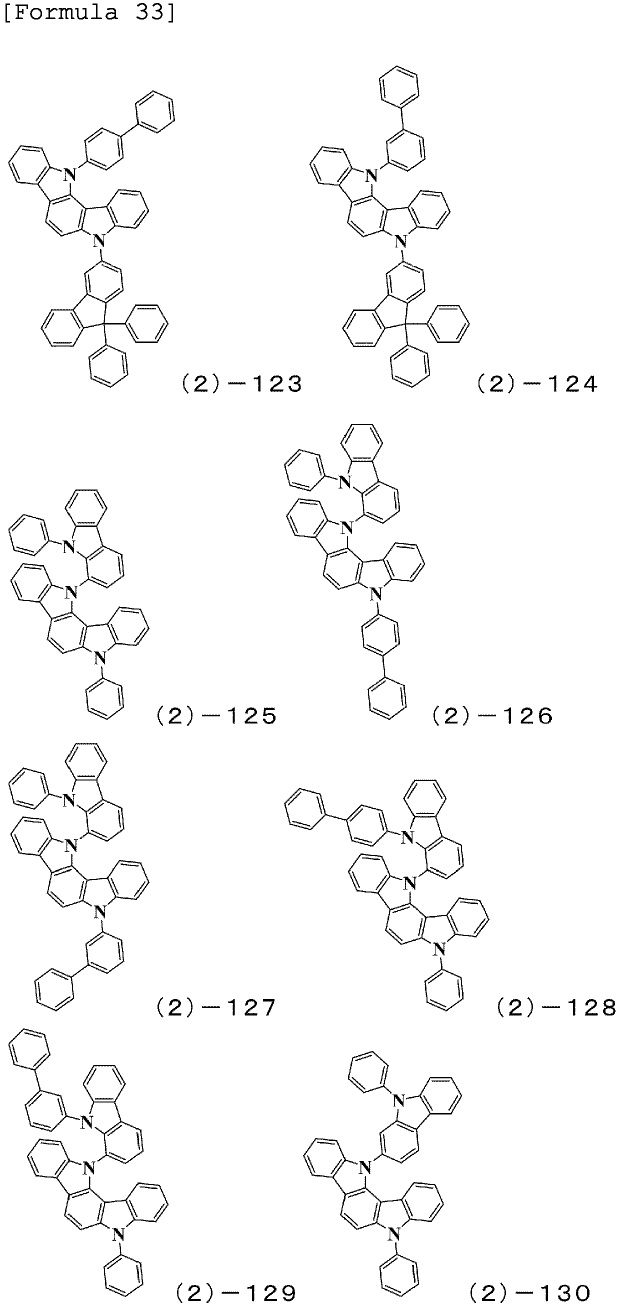

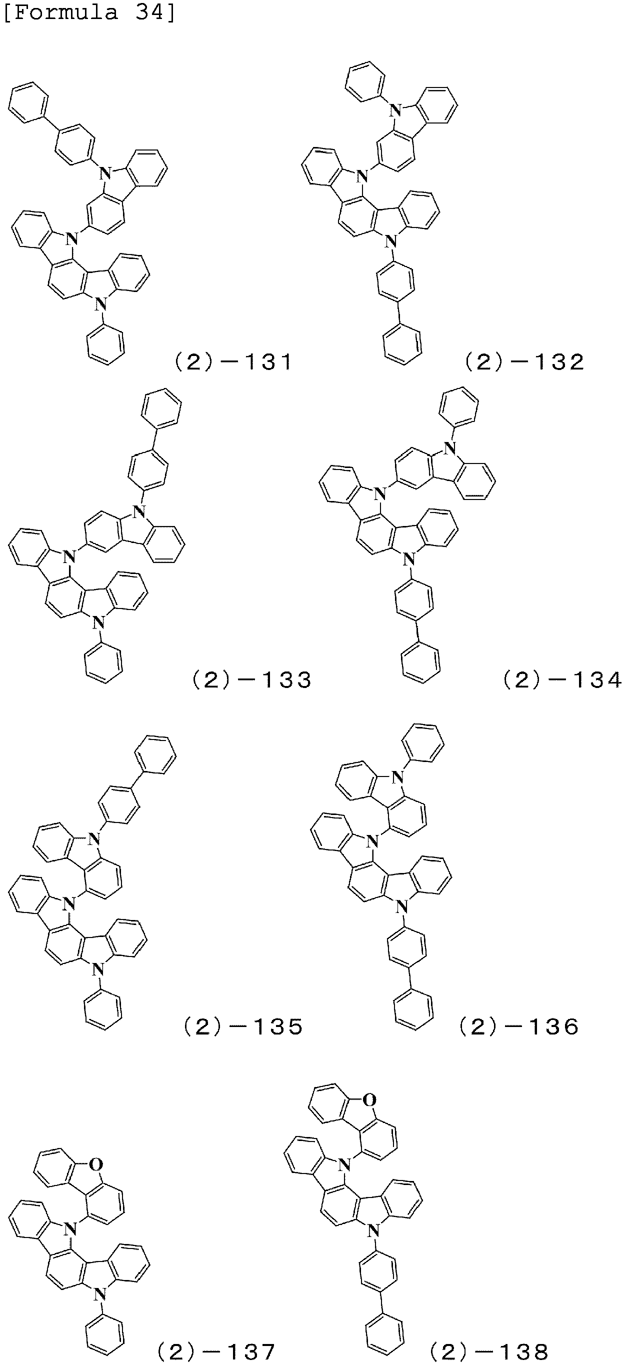

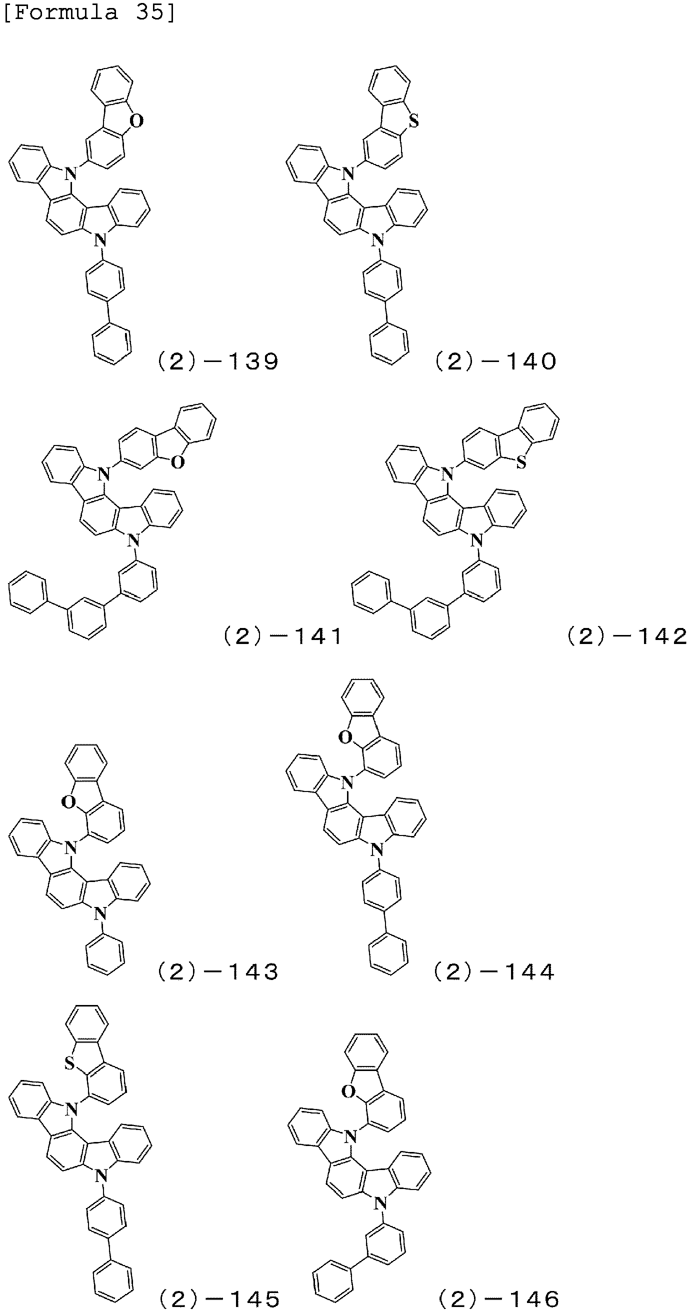

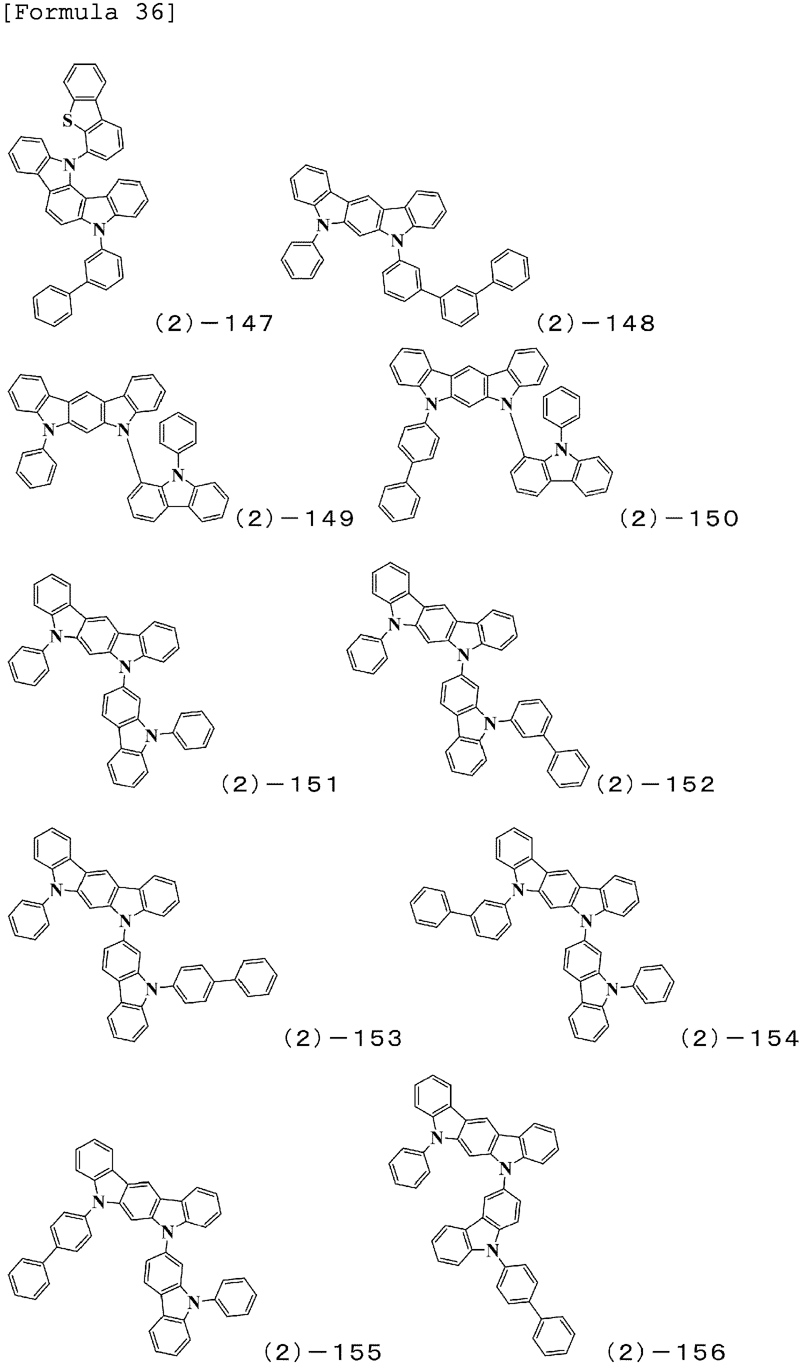

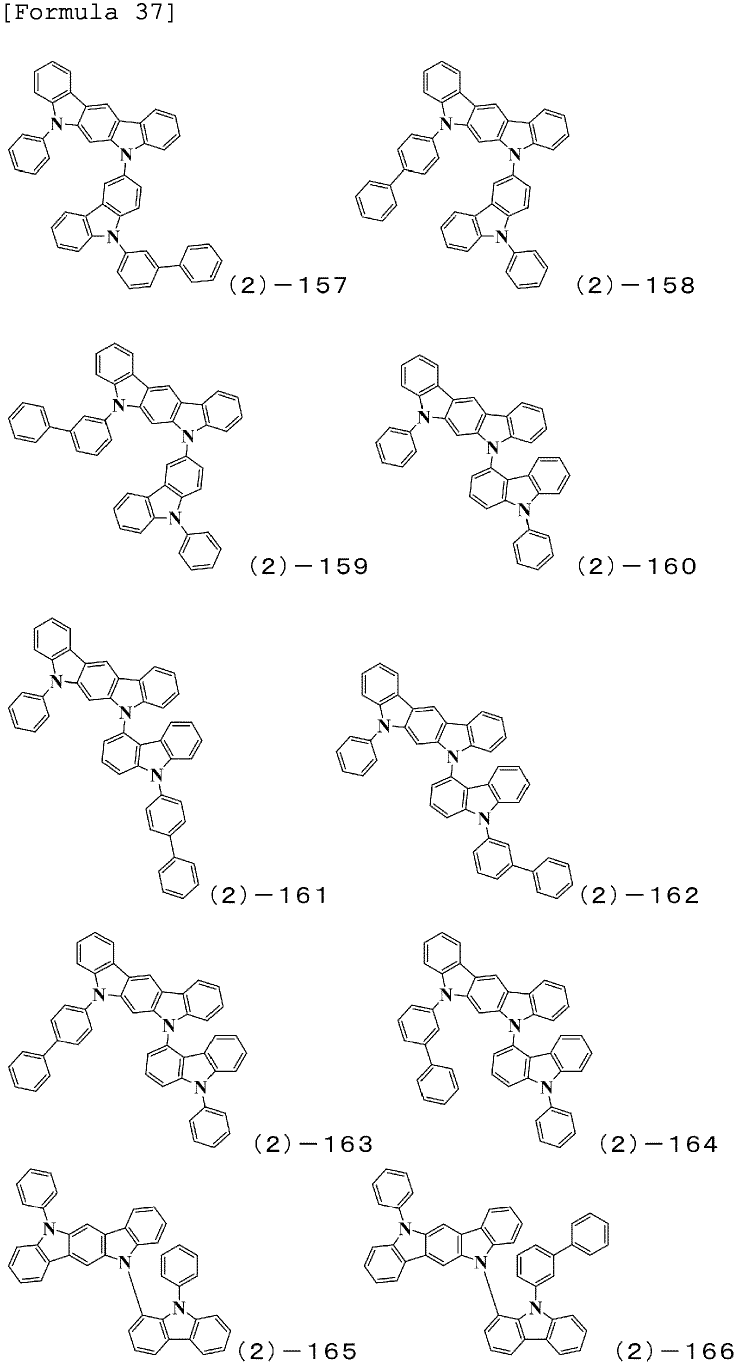

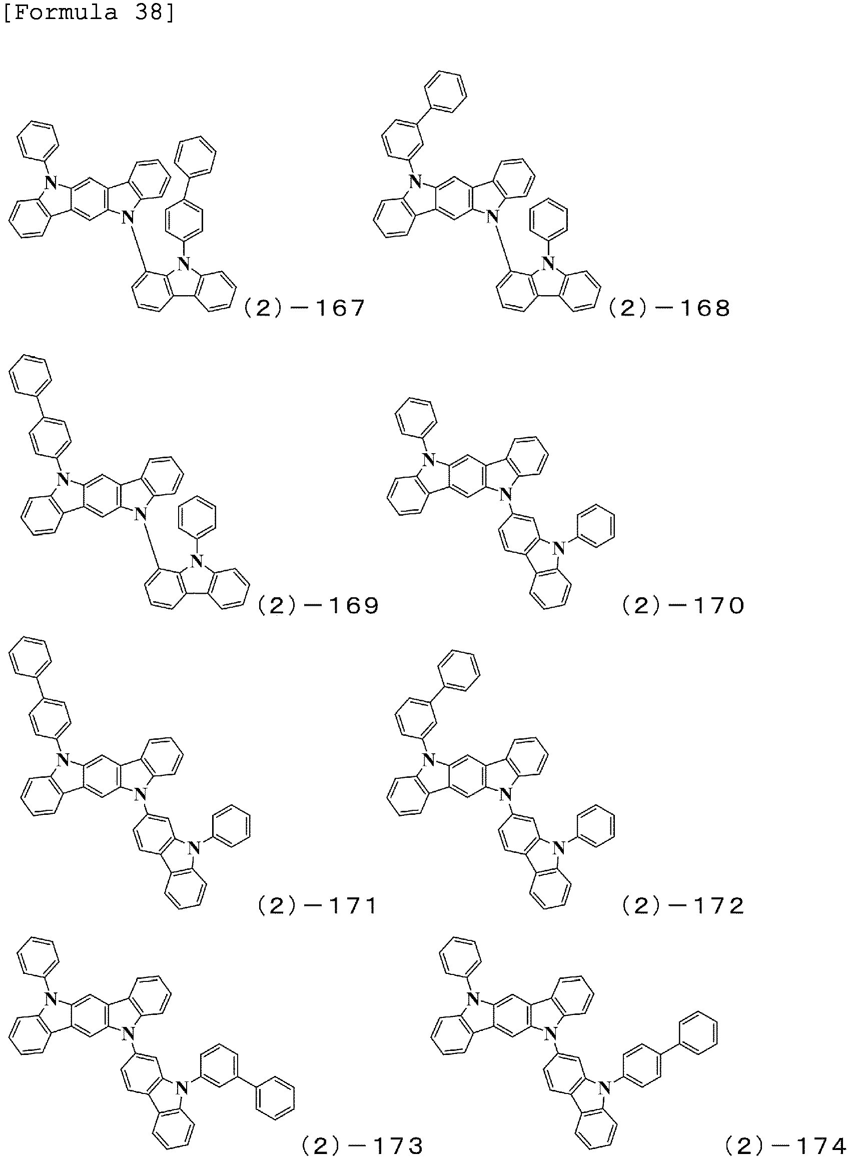

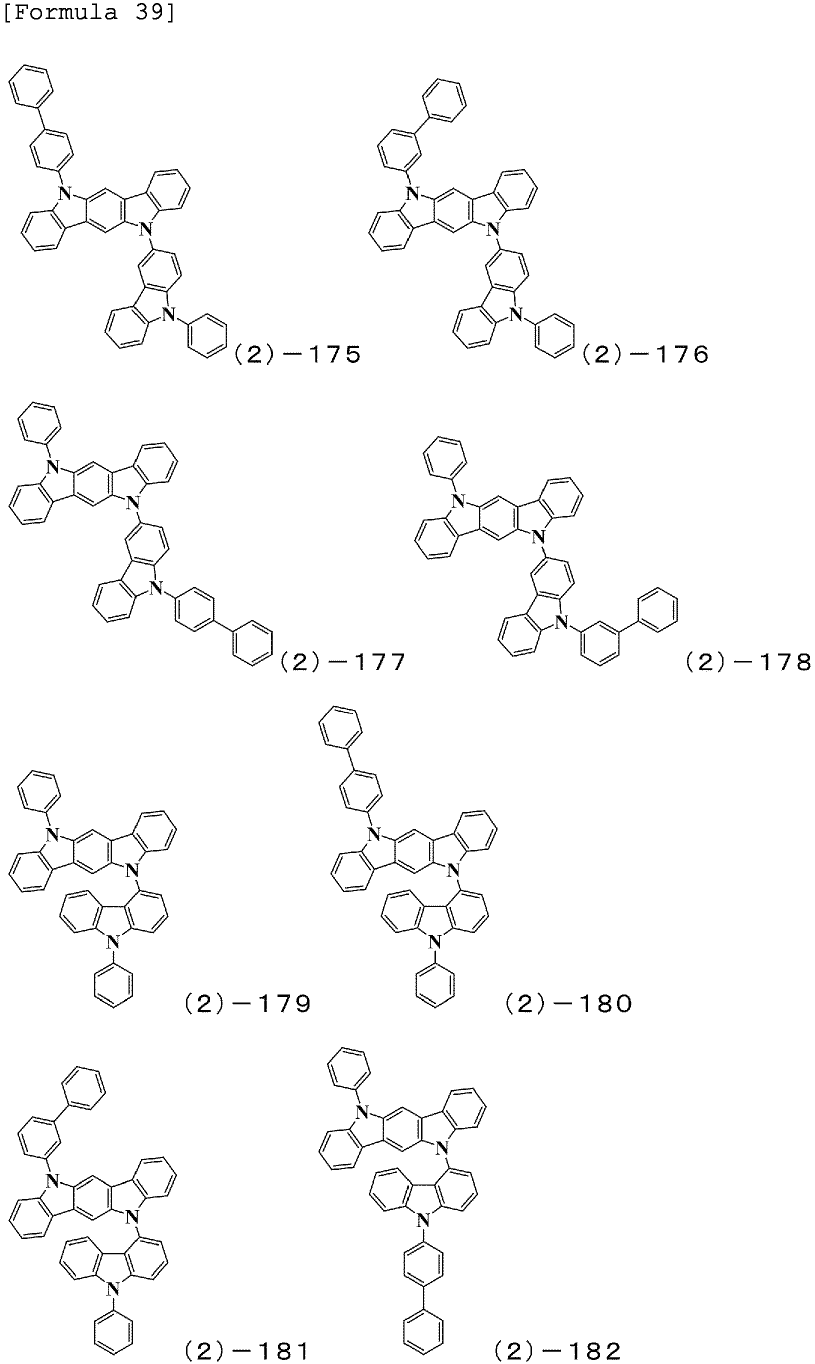

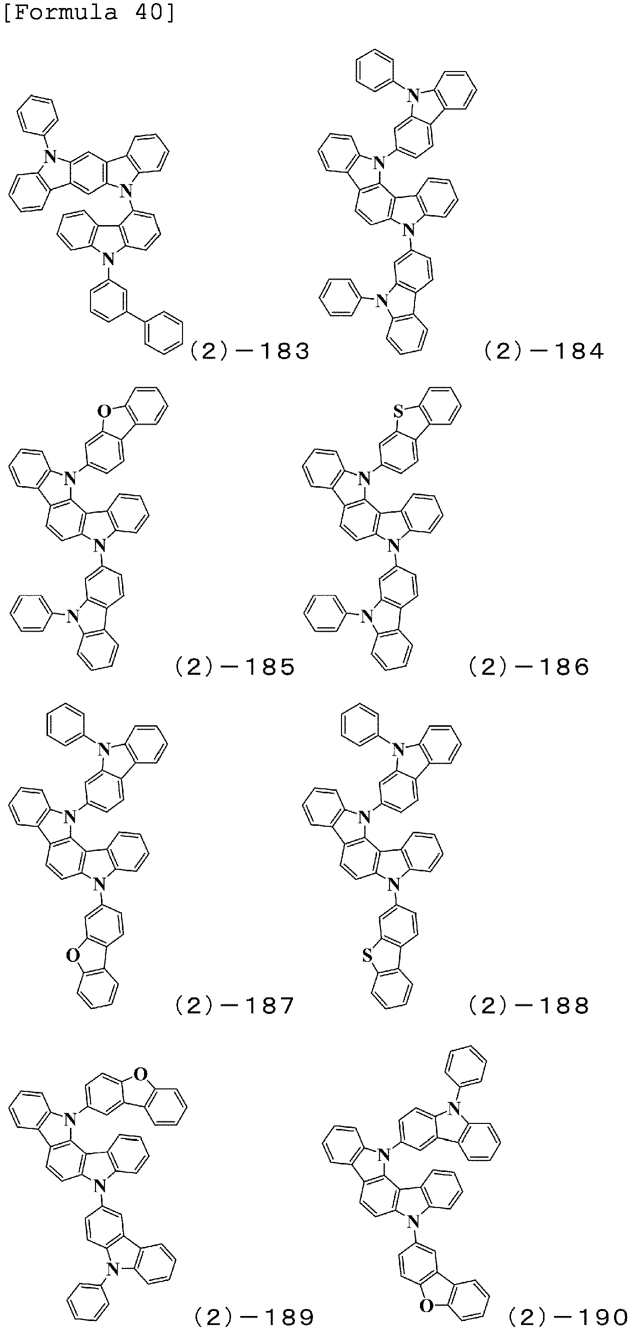

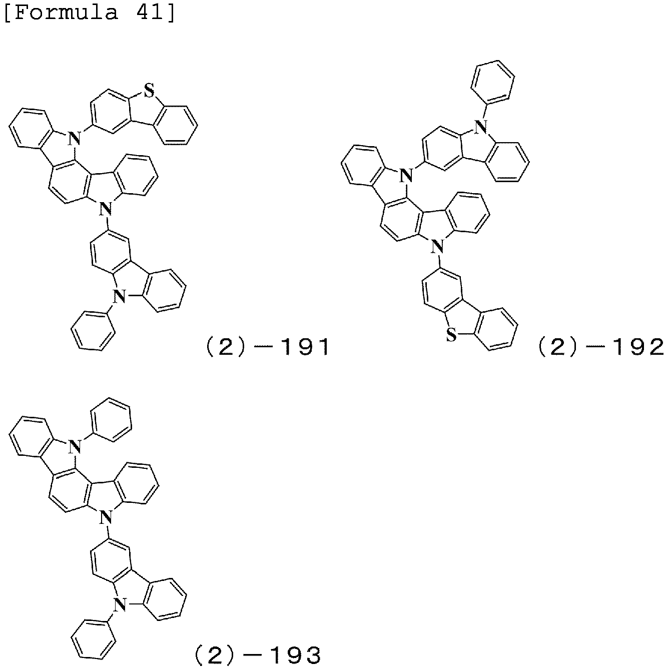

The organic EL device of the present invention has a light-emitting layer including the compound represented by the general formula (1), the compound represented by the general formula (2), and the dopant material, and the light-emitting layer may contain an aliphatic organic compound, an aromatic hydrocarbon compound, an aromatic heterocycle compound, an organic metal complex, and/or the like as other component(s). In the case of such other component(s) being contained, the proportion of such other compound(s) in total in the light-emitting layer is preferably less than 30 mass%, more preferably less than 10 mass%.

The proportion of the compound represented by the general formula (1) based on the compound represented by the general formula (1) and the compound represented by the general formula (2) in total is preferably 10 mass% or more and less than 70 mass%, more preferably 20 mass% or more and less than 60 mass%.

The light-emitting dopant material is preferably an organic metal complex containing at least one metal selected from the group consisting of ruthenium, rhodium, palladium, silver, rhenium, osmium, iridium, platinum and gold, or a fluorescence-emitting dopant material including thermally activated delayed fluorescence.

The light-emitting layer in the organic EL device of the present invention may be formed by vapor deposition of the first and second host materials and the dopant material, from a vapor deposition source, or may be formed by dissolution or dispersion of these materials in a solvent and a spin coating method, a bar coating method, a spraying method, an inkjet method, a printing method, or the like.

The light-emitting layer in the organic EL device of the present invention, if formed according to a vapor deposition method, can be formed by vapor deposition from different individual vapor deposition sources, but the light-emitting layer is preferably formed by obtaining a premixture by premixing materials before vapor deposition and vapor-depositing the premixture simultaneously from one vapor deposition source. In this case, the premixture may be mixed with a light-emitting dopant material necessary for formation of a light-emitting layer, or another host material to be used as necessary. However, when there is a large difference in temperatures to provide desired vapor pressure, vapor deposition may be performed from another vapor deposition source.

The light-emitting layer in the organic EL device of the present invention, if formed by a printing method, can be formed according to the printing method by dissolving or dispersing the compound represented by the general formula (1), the compound represented by the general formula (2) and the dopant material in a solvent to provide an ink for the light-emitting layer.

Next, the structure of the organic EL device of the present invention will be described by referring to the drawing, but the structure of the organic EL device of the present invention is not limited thereto.

Figure 1 is a cross-sectional view showing a structure example of an organic EL device generally used for the present invention, in which there are indicated a substrate 1, an anode 2, a hole injection layer 3, a hole transport layer 4, a light-emitting layer 5, an electron transport layer 6, and a cathode 7. The organic EL device of the present invention may have an exciton blocking layer adjacent to the light-emitting layer and may have an electron blocking layer between the light-emitting layer and the hole injection layer. The exciton blocking layer can be inserted into either of the anode side and the cathode side of the light-emitting layer and inserted into both sides at the same time. The organic EL device of the present invention has the anode, the light-emitting layer, and the cathode as essential layers, and preferably has a hole injection transport layer and an electron injection transport layer in addition to the essential layers, and further preferably has a hole blocking layer between the light-emitting layer and the electron injection transport layer. Note that the hole injection transport layer refers to either or both of a hole injection layer and a hole transport layer, and the electron injection transport layer refers to either or both of an electron injection layer and an electron transport layer.

A structure reverse to that of Figure 1 is applicable, in which a cathode 7, an electron transport layer 6, a light-emitting layer 5, a hole transport layer 4, and an anode 2 are laminated on a substrate 1 in this order. In this case, layers may be added or omitted as necessary.

### - Substrate -

The organic EL device of the present invention is preferably supported on a substrate. The substrate is not particularly limited, and those conventionally used in organic EL devices may be used, and substrates made of, for example, glass, a transparent plastic, or quartz may be used.

### - Anode -

Regarding an anode material for an organic EL device, it is preferable to use a material of a metal, an alloy, an electrically conductive compound, and a mixture thereof, each having a large work function (4 eV or more). Specific examples of such an electrode material include a metal such as Au, and a conductive transparent material such as CuI, indium tin oxide (ITO), SnO₂, and ZnO. In addition, an amorphous material such as IDIXO (In₂O₃-ZnO), which is capable of forming a transparent conductive film, may be used. Regarding the anode, such an electrode material is used to form a thin film by, for example, a vapor-deposition or sputtering method, and a desired shape pattern may be formed by a photolithographic method; or if the pattern accuracy is not particularly required (about 100 µm or more), a pattern may be formed via a desired shape mask when the electrode material is vapor-deposited or sputtered. Alternatively, when a coatable substance such as an organic conductive compound is used, a wet film formation method such as a printing method or a coating method may be used. For taking emitted light from the anode, it is desired to have a transmittance of more than 10%, and the sheet resistance for the anode is preferably several hundreds Ω/□ or less. The film thickness is selected usually within 10 to 1000 nm, preferably within 10 to 200 nm though depending on the material.

### - Cathode -

Meanwhile, regarding a cathode material, preferable to a material of a metal (an electron injection metal), an alloy, an electrically conductive compound, or a mixture thereof, each having a small work function (4 eV or less) are used. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, and a rare earth metal. Among these, from the viewpoint of the electron injectability and the durability against oxidation and the like, a mixture of an electron injection metal and a second metal which is a stable metal having a larger work function value is suitable, and examples thereof include a magnesium/silver mixture, a magnesium/aluminum mixture, a magnesium/indium mixture, an aluminum/aluminum oxide mixture, a lithium/aluminum mixture and aluminum. The cathode can be produced by forming a thin film by a method such as vapor-depositing or sputtering of such a cathode material. In addition, the sheet resistance of cathode is preferably several hundreds Ω/□ or less. The film thickness is selected usually within 10 nm to 5 µm, preferably within 50 to 200 nm. Note that for transmission of emitted light, if either one of the anode and cathode of the organic EL device is transparent or translucent, emission luminance is improved, which is convenient.

In addition, formation of a film of the above metal with a thickness of 1 to 20 nm on the cathode, followed by formation of a conductive transparent material described in the description on the anode thereon, enables production of a transparent or translucent cathode, and application of this enables production of a device wherein an anode and a cathode both have transmittance.

### - Light-emitting layer -

The light-emitting layer is a layer that emits light after excitons are generated when holes and electrons injected from the anode and the cathode, respectively, are recombined. The light-emitting layer contains the compound represented by the general formula (1), the compound represented by the general formula (2), and a light-emitting dopant material.

The compound represented by the general formula (1) and the compound represented by the general formula (2) are each preferably used as a host material of the light-emitting layer. Regarding the compound represented by the general formula (1), one kind thereof may be used, or two or more kinds thereof may be used. Similarly, regarding the compound represented by the general formula (2), one kind thereof may be used, or two or more kinds thereof may be used.

If necessary, one, or two or more known host materials may be used in combination; however, it is preferable that an amount thereof to be used be 50 mass% or less, preferably 25 mass% or less based on the host materials in total of the compound represented by the general formula (1) and the compound represented by the general formula (2).

When the compound represented by the general formula (1) and the compound represented by the general formula (2) are premixed and used, it is desirable that a difference in 50% weight reduction temperature (T₅₀) between the compounds be small in order to produce an organic EL device having favorable characteristics with high reproducibility. The 50% weight reduction temperature is a temperature at which the weight is reduced by 50% when the temperature is raised to 550°C from room temperature at a rate of 10°C/min in TG-DTA measurement under a nitrogen stream reduced pressure (1 Pa). It is considered that vaporization due to evaporation or sublimation the most vigorously occurs around this temperature.

The difference in 50% weight reduction temperatures of the compound represented by the general formula (1) and the compound represented by the general formula (2) is preferably within 30°C, more preferably within 20°C. Regarding a premixing method, a known method such as pulverization and mixing can be used, and it is desirable to mix them as uniformly as possible. The difference in 50% weight reduction temperatures is herein expressed by an absolute value.

In the case of use of a plurality of kinds of host materials, such respective host materials can be vapor-deposited from different vapor deposition sources or can be simultaneously vapor-deposited from one vapor deposition source by premixing the host materials before vapor deposition to provide a premixture.

The premixing method is desirably a method that can allow for mixing as uniformly as possible, and examples thereof include pulverization and mixing, a heating and melting method under reduced pressure or under an atmosphere of an inert gas such as nitrogen, and sublimation, but not limited thereto.

The forms of the hosts and the premixture thereof may be powdery, stick-shaped, or granular.

When a phosphorescent dopant material is used as a light-emitting dopant material, preferred is a phosphorescent dopant material including an organic metal complex containing at least one metal selected from ruthenium, rhodium, palladium, silver, rhenium, osmium, iridium, platinum and gold. Specifically, iridium complexes described in J. Am. Chem. Soc. 2001, 123, 4304 and Japanese Translation of PCT International Application Publication No. 2013-53051 are preferably used, but the phosphorescent dopant is not limited thereto.

Regarding the phosphorescent dopant material, only one kind thereof may be contained in the light-emitting layer, or two or more kinds thereof may be contained. A content of the phosphorescent dopant material is preferably 0.10 to 30 wt% and more preferably 1.0 to 20 wt% with respect to the host material.

The phosphorescent dopant material is not particularly limited, and specific examples thereof include the following.

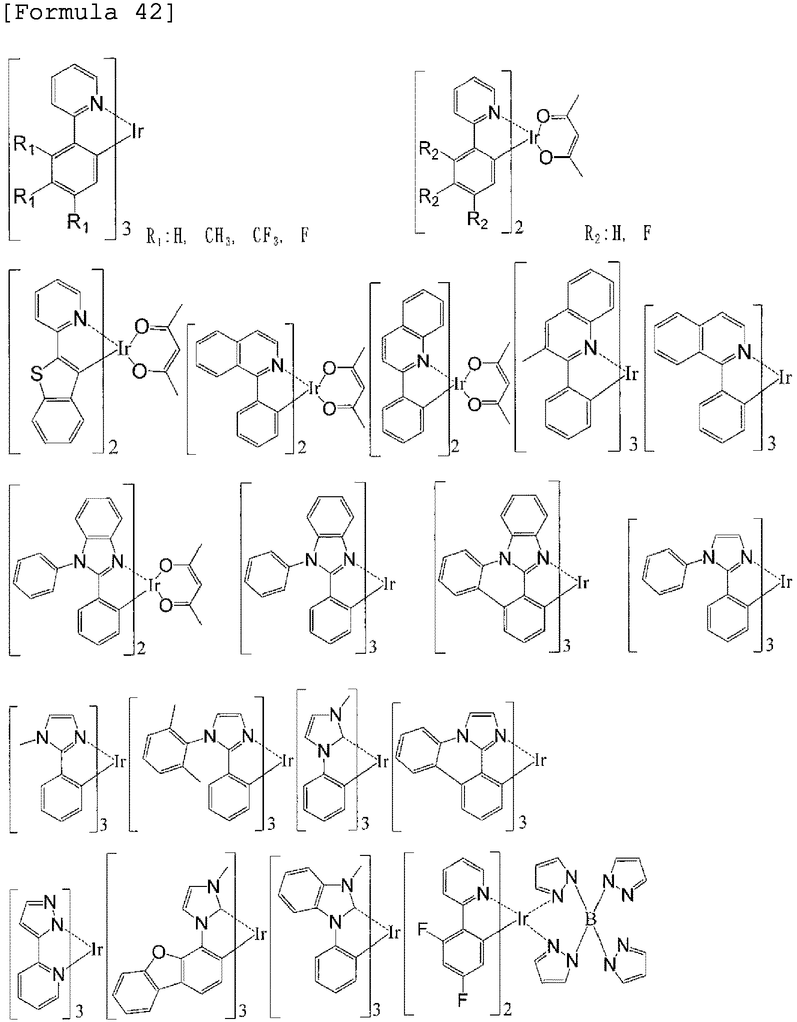

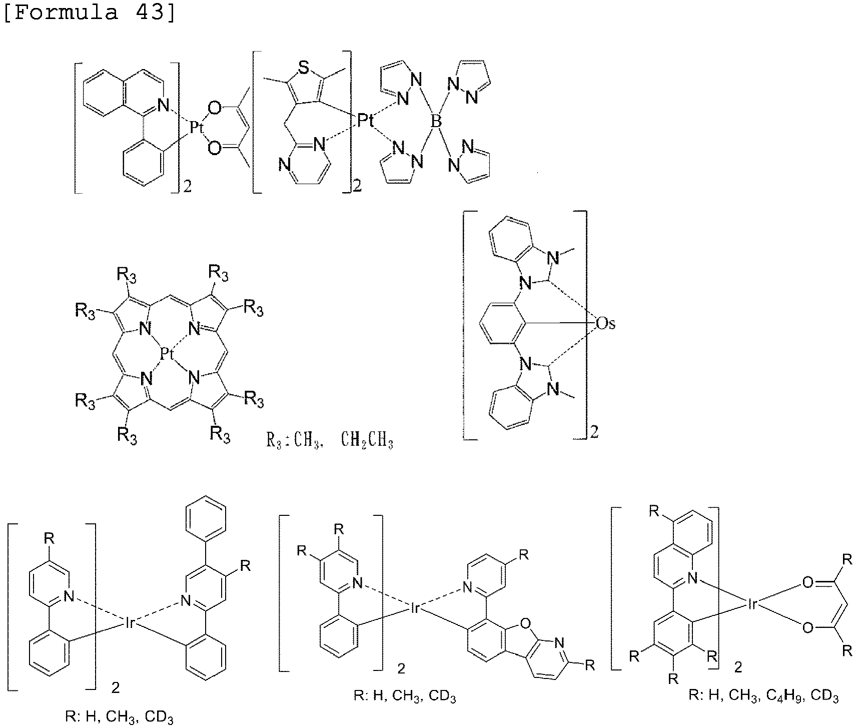

When a fluorescence-emitting dopant material is used as the light-emitting dopant material, the fluorescence-emitting dopant material is not particularly limited. Examples thereof include benzoxazole derivatives, benzothiazole derivatives, benzimidazole derivatives, styrylbenzene derivatives, polyphenyl derivatives, diphenylbutadiene derivatives, tetraphenyl butadiene derivatives, naphthalimido derivatives, coumarin derivatives, fused aromatic compounds, perinone derivatives, oxadiazole derivatives, oxazine derivatives, aldazine derivatives, pyrrolidine derivatives, cyclopentadiene derivatives, bisstyryl anthracene derivatives, quinacridone derivatives, pyrrolopyridine derivatives, thiadiazolopyridine derivatives, styrylamine derivatives, diketopyrrolopyrrole derivatives, aromatic dimethylidine compounds, metal complexes of 8-quinolinol derivatives or metal complexes of pyromethene derivatives, rare earth complexes, various metal complexes represented by transition metal complexes, polymer compounds such as polythiophene, polyphenylene, and polyphenylene vinylene, and organosilane derivatives. Preferred examples thereof include fused aromatic derivatives, styryl derivatives, diketopyrrolopyrrole derivatives, oxazine derivatives, pyromethene metal complexes, transition metal complexes, and lanthanoid complexes. More preferable examples thereof include naphthalene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo[a]naphthalene, hexacene, naphtho[2,1-f]isoquinoline, α-naphthaphenanthridine, phenanthrooxazole, quinolino[6,5-f]quinoline, and benzothiophanthrene. These may have an alkyl group, an aryl group, an aromatic heterocyclic group, or a diarylamino group as a substituent.

Regarding the fluorescence-emitting dopant material, only one kind thereof may be contained in the light-emitting layer, or two or more kinds thereof may be contained. A content of the fluorescence-emitting dopant material is preferably 0.10% to 20% and more preferably 1.0% to 10% with respect to the host material.

When a thermally activated delayed fluorescence-emitting dopant material is used as the light-emitting dopant material, the thermally activated delayed fluorescence-emitting dopant material is not particularly limited. Examples thereof include: metal complexes such as a tin complex and a copper complex; indolocarbazole derivatives described in WO2011/070963; cyanobenzene derivatives and carbazole derivatives described in Nature 2012, 492, 234; and phenazine derivatives, oxadiazole derivatives, triazole derivatives, sulfone derivatives, phenoxazine derivatives, and acridine derivatives described in Nature Photonics 2014, 8,326.

The thermally activated delayed fluorescence-emitting dopant material is not particularly limited, and specific examples thereof include the following.

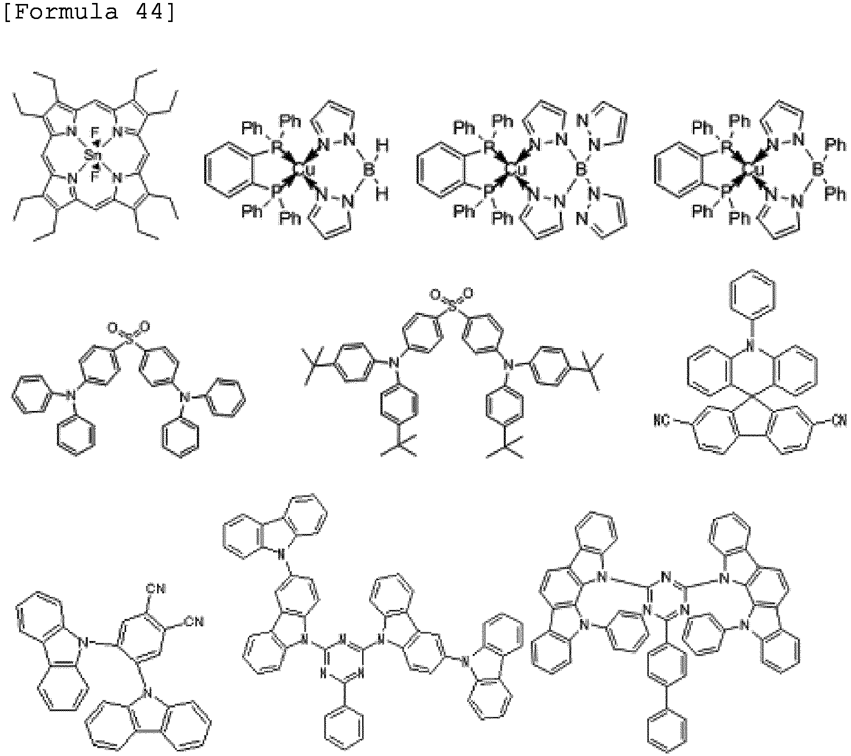

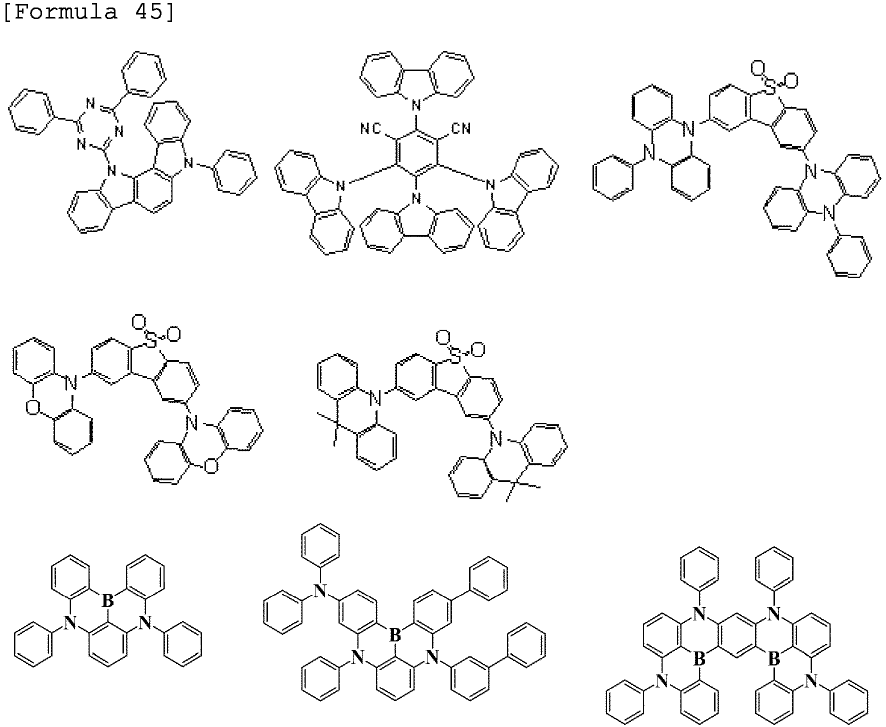

Regarding the thermally activated delayed fluorescence-emitting dopant material, only one kind thereof may be contained in the light-emitting layer, or two or more kinds thereof may be contained. In addition, the thermally activated delayed fluorescence-emitting dopant material may be used by mixing with a phosphorescent dopant material and a fluorescence-emitting dopant material. A content of the thermally activated delayed fluorescence-emitting dopant material is preferably 0.10 mass% to 50 mass% and more preferably 1.0 mass% to 30 mass% with respect to the host material.

### - Injection Layer -

The injection layer is a layer that is provided between an electrode and an organic layer in order to lower a driving voltage and improve emission luminance, and includes a hole injection layer and an electron injection layer, and may be present between the anode and the light-emitting layer or the hole transport layer, and between the cathode and the light-emitting layer or the electron transport layer. The injection layer can be provided as necessary.

### - Hole Blocking Layer -

The hole blocking layer has a function of the electron transport layer in a broad sense, and is made of a hole blocking material having a function of transporting electrons and a significantly low ability to transport holes, and can block holes while transporting electrons, thereby improving a probability of recombining electrons and holes in the light-emitting layer.

For the hole blocking layer, a known hole blocking layer material can be used, but it is preferred for the layer to contain the compound represented by the general formula (1).

### - Electron Blocking Layer -

The electron blocking layer has a function of a hole transport layer in a broad sense and blocks electrons while transporting holes, thereby enabling a probability of recombining electrons and holes in the light-emitting layer to be improved.

Regarding the material of the electron blocking layer, a known electron blocking layer material can be used and a material of the hole transport layer to be described below can be used as necessary. A film thickness of the electron blocking layer is preferably 3 to 100 nm, and more preferably 5 to 30 nm.

### - Exciton Blocking Layer -

The exciton blocking layer is a layer for preventing excitons generated by recombination of holes and electrons in the light-emitting layer from being diffused in a charge transport layer, and insertion of this layer allows excitons to be efficiently confined in the light-emitting layer, enabling the luminous efficiency of the device to be improved. The exciton blocking layer can be inserted, in a device having two or more light-emitting layers adjacent to each other, between two adjacent light-emitting layers.

Regarding the material of the exciton blocking layer, a known exciton blocking layer material can be used. Examples thereof include 1,3-dicarbazolyl benzene (mCP) and bis(2-methyl-8-quinolinolato)-4-phenylphenolato aluminum (III) (BAlq).

### - Hole Transport Layer -

The hole transport layer is made of a hole transport material having a function of transporting holes, and the hole transport layer can be provided as a single layer or a plurality of layers.

The hole transport material has either hole injection, transport properties or electron barrier properties, and may be an organic material or an inorganic material. For the hole transport layer, any one selected from conventionally known compounds can be used. Examples of such a hole transport material include porphyrin derivatives, arylamine derivatives, triazole derivatives, oxadiazole derivatives, imidazole derivatives, polyarylalkane derivatives, pyrazoline derivatives and pyrazolone derivatives, phenylenediamine derivatives, arylamine derivatives, amino-substituted chalcone derivatives, oxazole derivatives, styryl anthracene derivatives, fluorenone derivatives, hydrazone derivatives, stilbene derivatives, silazane derivatives, an aniline copolymer, and a conductive polymer oligomer, and particularly a thiophene oligomer. Use of porphyrin derivatives, arylamine derivatives, or styrylamine derivatives preferred. Use of arylamine compounds is more preferred.

### - Electron Transport Layer -

The electron transport layer is made of a material having a function of transporting electrons, and the electron transport layer can be provided as a single layer or a plurality of layers.

The electron transport material (which may also serve as a hole blocking material) may have a function of transferring electrons injected from the cathode to the light-emitting layer. For the electron transport layer, any one selected from conventionally known compounds can be used, and examples thereof include polycyclic aromatic derivatives such as naphthalene, anthracene, and phenanthroline, tris(8-quinolinolato)aluminum(III) derivatives, phosphine oxide derivatives, nitro-substituted fluorene derivatives, diphenylquinone derivatives, thiopyrandioxide derivatives, carbodiimide, fluorenylidene methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. In addition, a polymer material in which the above material is introduced into a polymer chain or the above material is used for a main chain of a polymer can be used.

### Examples

Hereafter, the present invention will be described in detail by referring to Examples, but the present invention is not limited these Examples and can be implemented in various forms without departing from the gist thereof.

Hereinafter, compounds used in Examples and Comparative Examples are shown below.

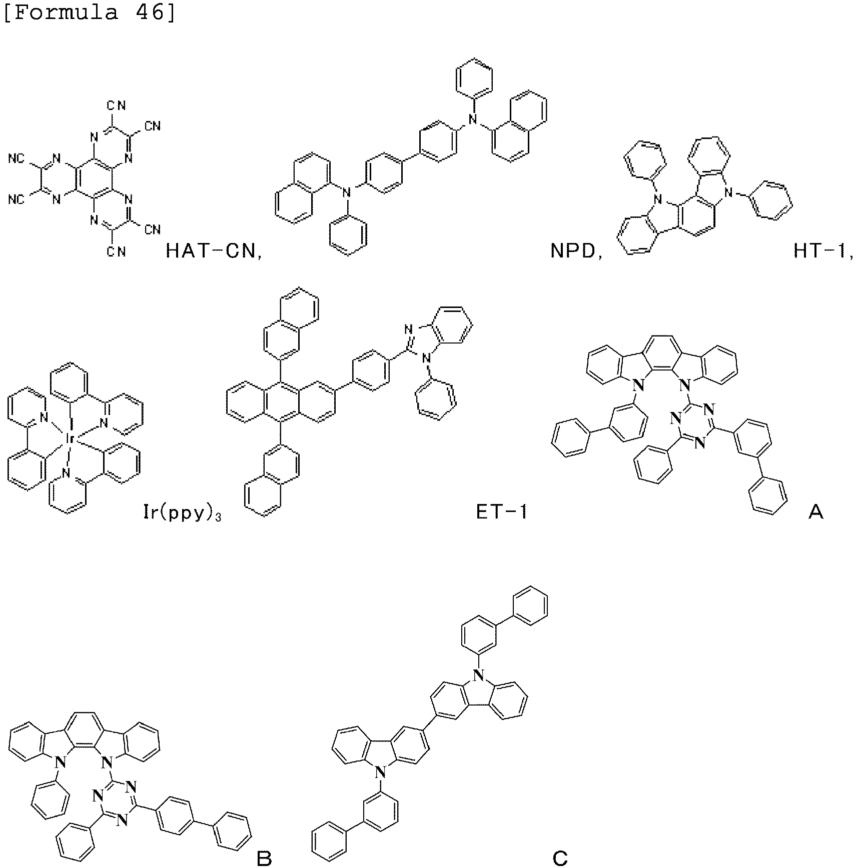

Table 1 shows the 50% weight reduction temperatures (T₅₀) of compounds (1)-1 and (1)-3, and the like, as the above compound represented by the general formula (1), and also the compound represented by the general formula (2) and compounds A and C. The 50% weight reduction temperature corresponds to a temperature at which the weight is reduced by 50% when the temperature is raised to 550°C from room temperature at a rate of 10°C/min in TG-DTA measurement under a nitrogen stream reduced pressure (1 Pa).

**[Table 1]**

| Compound | T₅₀[°C] |
|---|---|
| (1)-1 | 290 |
| (1)-2 | 310 |
| (1)-3 | 280 |
| (1)-31 | 319 |
| (2)-6 | 265 |
| (2)-82 | 300 |
| (2)-84 | 308 |
| Compound A | 271 |
| Compound C | 312 |

### Example 1

On a glass substrate on which an anode made of ITO with a film thickness of 110 nm was formed, respective thin films were laminated by a vacuum evaporation method at a degree of vacuum of 4.0×10⁻⁵ Pa. First, HAT-CN was formed with a thickness of 25 nm as a hole injection layer on ITO, and next, NPD was formed with a thickness of 30 nm as a hole transport layer. Next, HT-1 was formed with a thickness of 10 nm as an electron blocking layer. Then, compound (1)-1 as a first host material, compound (2)-6 as a second host material and Ir(ppy)₃ as a light-emitting dopant material were co-vapor-deposited from different vapor deposition sources, respectively, to form a light-emitting layer with a thickness of 40 nm. In this case, co-vapor deposition was performed under vapor deposition conditions such that the concentration of Ir(ppy)₃ was 10 mass%, the concentration of a mixed host composed of the first host material and the second host material was 90 mass% and the mass ratio between the first host material and the second host material was 30:70. Next, ET-1 was formed with a thickness of 20 nm as an electron transport layer. Further, LiF was formed with a thickness of 1 nm as an electron injection layer on the electron transport layer. Finally, Al was formed with a thickness of 70 nm as a cathode on the electron injection layer to produce an organic EL device.

### Examples 2 to 6

Organic EL devices were produced in the same manner as in Example 1 except that compounds shown in Table 2 were used as the first host material and the second host material instead of those of Example 1.

### Examples 7 to 13

Organic EL devices were produced in the same manner as in Example 1 except that, in Example 1, the first host material and the second host material used were compounds described in Table 2 and co-vapor deposition was performed such that the mass ratio between the first host material and the second host material was each value described in Table 2.

### Examples 14 to 18

Organic EL devices were produced in the same manner as in Example 1 except that, in Example 1, the first host material and the second host material used were compounds described in Table 2, and the first host and the second host were mixed in advance at a mass ratio shown in Table 2 to prepare a premixture and the premixture was vapor-deposited from one vapor deposition source.

### Comparative Examples 1 to 4

Organic EL devices were produced in the same manner as in Example 1 except that a compound described in Table 2 was used alone as the host material in Example 1. The thickness of the light-emitting layer and the concentration of the light-emitting dopant were the same as those in Example 1.

### Comparative Examples 5 to 10

Organic EL devices were produced in the same manner as in Example 1 except that, in Example 1, the first host material and the second host material used were compounds described in Table 2.

### Comparative Examples 11 to 13

Organic EL devices were produced in the same manner as in Example 1 except that, in Example 1, the first host material and the second host material used were compounds described in Table 2, and the first host material and the second host material were mixed in advance to prepare a premixture and the premixture was vapor-deposited from one vapor deposition source.

Evaluation results of the produced organic EL devices are shown in Table 2.

In the Table, the luminance, the driving voltage, and power efficiency are values at a driving current of 20 mA/cm², and they exhibit initial characteristics.

LT70 is a time period needed for the initial luminance to be reduced to 70% thereof, and it represents lifetime characteristics.

**[Table 2]**

| | First host material | Second host material | Mixing ratio (mass %) | Driving voltage (V) | Luminance (cd/m2) | Power efficiency (Im/W) | LT70 (h) |
|---|---|---|---|---|---|---|---|
| Example 1 | (1)-1 | (2)-6 | 30/70 | 4.3 | 11900 | 43.4 | 1420 |
| Example 2 | (1)-1 | (2)-82 | 30/70 | 4.1 | 11600 | 44.7 | 2200 |
| Example 3 | (1)-1 | (2)-84 | 30/70 | 4.2 | 11800 | 44.2 | 2430 |
| Example 4 | (1)-2 | (2)-84 | 30/70 | 4.3 | 11500 | 42.4 | 2120 |
| Example 5 | (1)-3 | (2)-154 | 30/70 | 4.2 | 11600 | 43.0 | 1880 |
| Example 6 | (1)-16 | (2)-82 | 30/70 | 4.3 | 12000 | 43.9 | 2290 |
| Example 7 | (1)-1 | (2)-82 | 40/60 | 3.9 | 11700 | 47.2 | 1950 |
| Example 8 | (1)-11 | (2)-84 | 40/60 | 3.9 | 11600 | 47.3 | 1640 |
| Example 9 | (1)-31 | (2)-154 | 40/60 | 4.0 | 11400 | 44.5 | 1720 |
| Example 10 | (1)-1 | (2)-82 | 50/50 | 3.7 | 11800 | 50.8 | 1650 |
| Example 11 | (1)-2 | (2)-84 | 50/50 | 3.8 | 11800 | 48.6 | 1760 |
| Example 12 | (1)-11 | (2)-84 | 50/50 | 3.6 | 11500 | 49.6 | 1460 |
| Example 13 | (1)-31 | (2)-171 | 50/50 | 3.6 | 11200 | 48.9 | 1390 |
| Example 14 | (1)-1 | (2)-82 | 30/70 | 4.0 | 11700 | 45.5 | 2370 |
| Example 15 | (1)-2 | (2)-84 | 30/70 | 4.3 | 11500 | 42.3 | 2160 |
| Example 16 | (1)-1 | (2)-82 | 40/60 | 3.8 | 11800 | 48.8 | 2010 |
| Example 17 | (1)-2 | (2)-84 | 40/60 | 4.0 | 11600 | 46.1 | 1850 |
| Example 18 | (1)-1 | (2)-82 | 50/50 | 3.7 | 11900 | 51.1 | 1790 |
| Comp. Example 1 | (1)-1 | | - | 3.1 | 8900 | 45.1 | 1150 |
| Comp. Example 2 | (1)-7 | | - | 3.8 | 7500 | 31.0 | 200 |
| Comp. Example 3 | (1)-16 | | - | 3.3 | 9500 | 45.2 | 1220 |
| Comp. Example 4 | | (2)-6 | - | 6.1 | 7800 | 20.1 | 250 |
| Comp. Example 5 | Compound A | (2)-82 | 30/70 | 4.5 | 11400 | 40.2 | 1150 |
| Comp. Example 6 | (1)-1 | Compound C | 30/70 | 4.5 | 11600 | 40.8 | 1310 |
| Comp. Example 7 | Compound A | (2)-6 | 30/70 | 4.6 | 11600 | 39.4 | 950 |
| Comp. Example 8 | Compound A | (2)-82 | 40/60 | 4.4 | 11500 | 41.2 | 1180 |
| Comp. Example 9 | Compound B | (2)-193 | 40/60 | 4.3 | 10600 | 38.4 | 1450 |
| Comp. Example 10 | Compound A | (2)-171 | 50/50 | 3.9 | 10500 | 42.3 | 990 |
| Comp. Example 11 | Compound A | (2)-6 | 30/70 | 4.4 | 11200 | 39.8 | 1110 |
| Comp. Example 12 | (1)-31 | Compound C | 40/60 | 4.3 | 11400 | 42.1 | 880 |
| Comp. Example 13 | (1)-2 | Compound C | 40/60 | 4.2 | 11500 | 43.0 | 1030 |

It can be seen from comparison between Examples of the present invention and Comparative Examples 1 to 4 in Table 2 that lifetime characteristics are remarkably enhanced by use of a mixed host. It can be seen from comparison between Examples of the present invention and Comparative Examples 5 to 13 that an organic EL device to which a combination of the host materials disclosed in the present invention is applied satisfies a low driving voltage and a high luminance and is significantly improved in power efficiency even in both the cases of co-vapor deposition and premixing. It can be seen that lifetime characteristics are also very excellent.

## Claims

1. An organic electroluminescent device having a plurality of organic layers between an anode and a cathode, wherein the organic layers have at least one light-emitting layer, the light-emitting layer comprises two host materials different from each other, and a dopant material, and one of the host materials is a compound represented by the following general formula (1) and the other of the host materials is a compound represented by the following general formula (2): 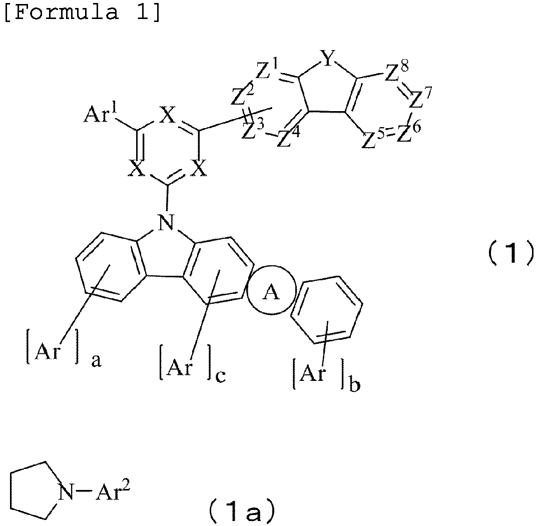 wherein a ring A is a heterocycle represented by formula (1a) and the ring A is fused at any position of an adjacent ring;
X represents N or C-Ar', wherein at least one X represents N;
Y represents O, S, N-Ar³, or C-Ar⁴Ar⁵;
any one of Z¹ to Z⁴ represents a carbon atom binding to a 6-membered ring containing X, the others and Z⁵ to Z⁸ each independently represent C-Ar' or N, and Ar', when present in a plurality, may be the same or different;
Ar, Ar' and Ar¹ to Ar⁵ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other, provided that, when the group has a hydrogen atom, the hydrogen atom is optionally replaced with deuterium or halogen; and
a and b represent the number of substitutions and each independently represent an integer of 1 to 4; and c represents the number of substitutions and represents an integer of 1 to 2; 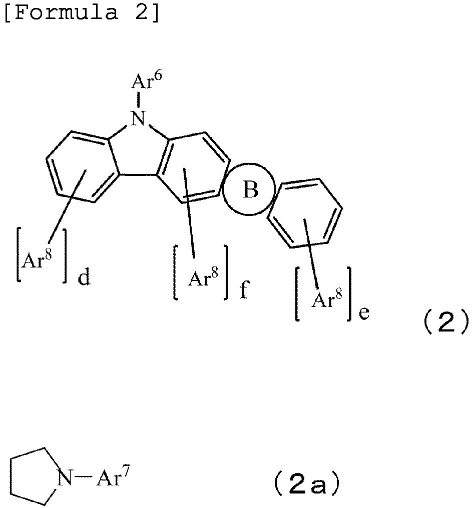 wherein a ring B is a heterocycle represented by formula (2a) and the ring B is fused at any position of an adjacent ring;
Ar⁶ and Ar⁷ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other, provided that, when the group has a hydrogen atom, the hydrogen atom is optionally replaced with deuterium or halogen;
d and e represent the number of substitutions and each represent an integer of 1 to 4; and f represents the number of substitutions and represents an integer of 1 to 2; and
each Ar⁸ independently represents hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other.

2. The organic electroluminescent device according to claim 1, wherein in the general formula (1), any of Z¹ or Z² is linked to a 6-membered ring containing X.

3. The organic electroluminescent device according to claim 1 or 2, wherein in the general formula (1), Y represents O or S.

4. The organic electroluminescent device according to claim 1, wherein the general formula (1) is represented by any of the following general formulas (4) to (8): 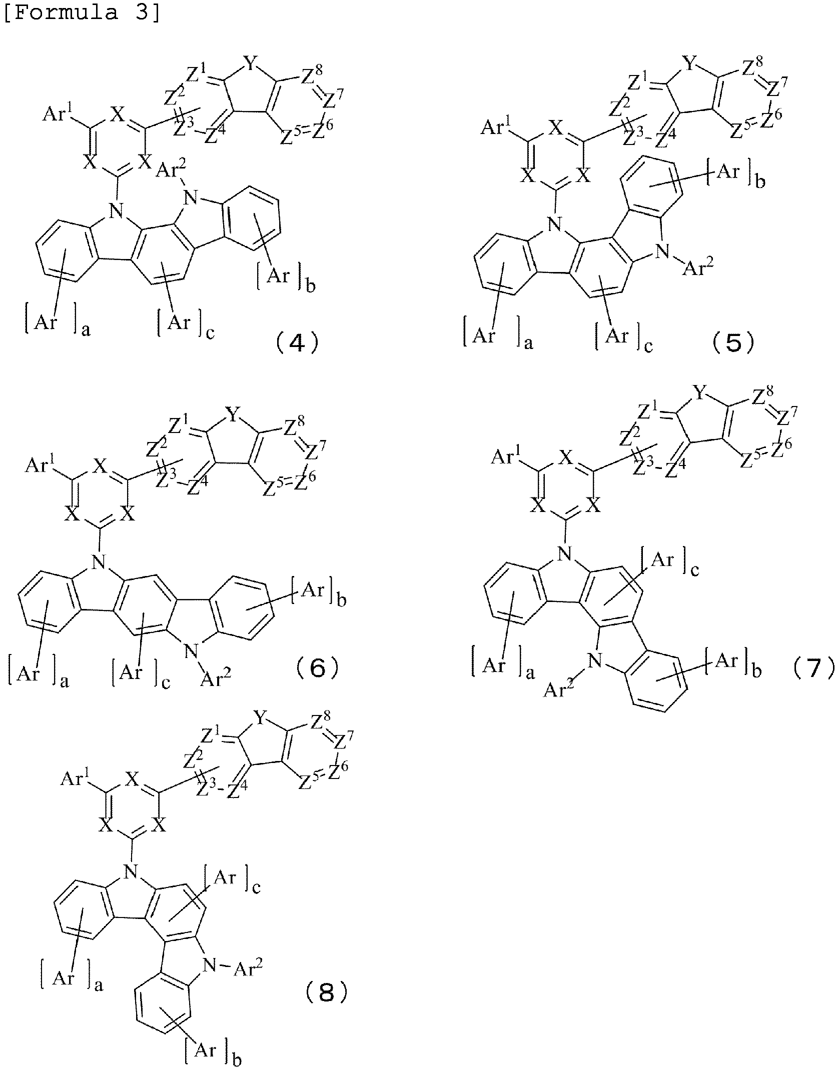 wherein X, Y, Ar, Ar¹, Ar², Z¹ to Z⁸, a, b, and c have the same meaning as in the general formula (1).

5. The organic electroluminescent device according to claim 4, wherein in the general formulas (4) to (8), Z² is linked to a 6-membered ring containing X.

6. The organic electroluminescent device according to claim 5, wherein in the general formulas (4) to (8), Ar¹ and Ar² each represent a substituted or unsubstituted aromatic hydrocarbon group having 6 to 10 carbon atoms, or a linked aromatic group in which two to five of these aromatic rings are linked to each other.

7. The organic electroluminescent device according to any one of claims 1 to 6, wherein the general formula (2) is represented by any of the following general formulas (9) to (13): 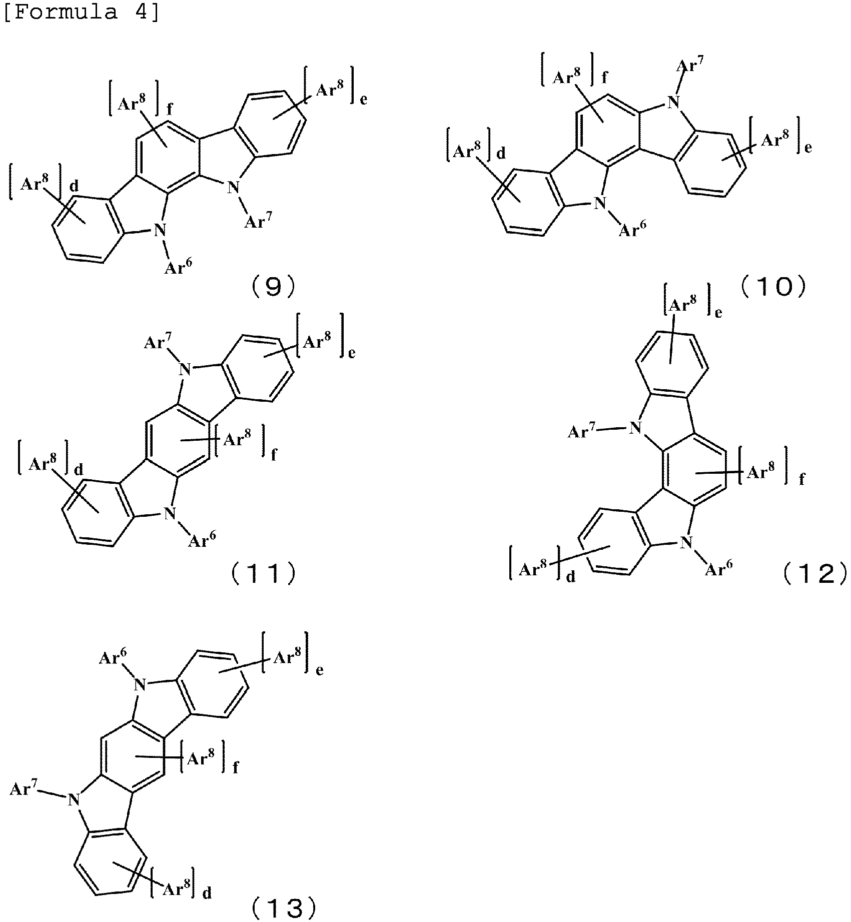 wherein Ar⁸, d, e, f, Ar⁶ and Ar⁷ have the same meaning as in the formula (2).

8. The organic electroluminescent device according to claim 7, wherein in the general formulas (9) to (13), Ar⁶ and Ar⁷ each represent a substituted or unsubstituted aromatic hydrocarbon group having 6 to 10 carbon atoms, a substituted or unsubstituted aromatic heterocyclic group having 3 to 17 carbon atoms, or a linked aromatic group in which two to five of these aromatic rings are linked to each other, and at least one of Ar⁶ and Ar⁷ contains one or more aromatic heterocycles having 6 to 17 carbon atoms.

9. The organic electroluminescent device according to any one of claims 1 to 8, wherein the dopant material is a phosphorescent dopant material or a fluorescent dopant material emitting thermally activated delayed fluorescence.

10. A mixed composition comprising a compound represented by the following general formula (1) and a compound represented by the following general formula (2) : 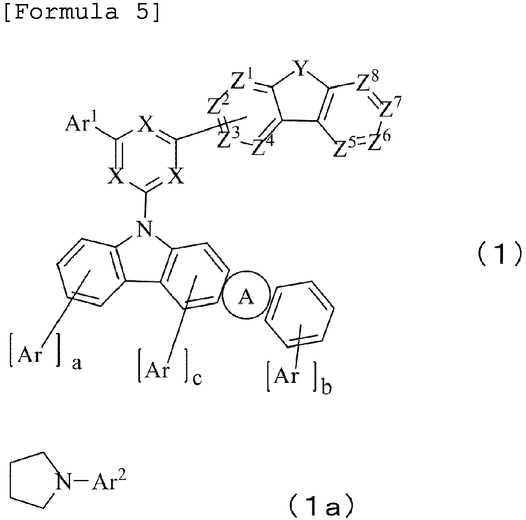 wherein a ring A is a heterocycle represented by formula (1a) and the ring A is fused at any position of an adjacent ring;
X represents N or C-Ar', wherein at least one X represents N;
Y represents O, S, N-Ar³, or C-Ar⁴Ar⁵;
any one of Z¹ to Z⁴ represents a carbon atom binding to a 6-membered ring containing X, the others and Z⁵ to Z⁸ each independently represent C-Ar' or N, and Ar', when present in a plurality, may be the same or different;
Ar, Ar' and Ar¹ to Ar⁵ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other, provided that, when the group has a hydrogen atom, the hydrogen atom is optionally replaced with deuterium or halogen; and
a and b represent the number of substitutions and each independently represent an integer of 1 to 4; and c represents the number of substitutions and represents an integer of 1 to 2; 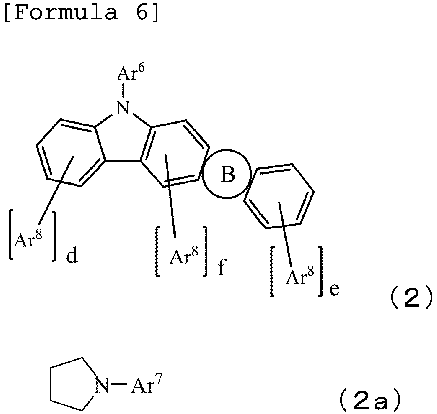 wherein a ring B is a heterocycle represented by formula (2a) and the ring B is fused at any position of an adjacent ring;
Ar⁶ and Ar⁷ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other, provided that, when the group has a hydrogen atom, the hydrogen atom is optionally replaced with deuterium or halogen;
d and e represent the number of substitutions and each represent an integer of 1 to 4; and f represents the number of substitutions and represents an integer of 1 to 2; and
each Ar⁸ independently represents hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 30 carbon atoms, a substituted or unsubstituted aromatic heterocyclic group having 3 to 17 carbon atoms, or a linked aromatic group in which two to five of these aromatic rings are linked to each other.

11. The mixed composition according to claim 10, wherein a difference in 50% weight reduction temperatures of the compound represented by the general formula (1) and the compound represented by the general formula (2) is within 20°C.

12. A method for producing an organic electroluminescent device, comprising producing a light-emitting layer by use of the mixed composition according to claim 10 or 11.
